# EUROPÄISCHE PATENTSCHRIFT

(11) **EP 1 117 726 B1**
(45) Veröffentlichungstag und Bekanntmachung des Hinweises auf die Patenterteilung: **03.05.2006**
(21) Anmeldenummer: 99944116.5
(22) Anmeldetag: 25.08.1999
(51) Int. Cl.: C08G 18/28, C08G 18/67, C08F 290/06

(54) **HAARBEHANDLUNGSMITTEL AUF BASIS RADIKALISCH POLYMERISIERBARER, SILOXANGRUPPENHALTIGER URETHAN(METH)ACRYLATE UND DEREN POLYMERISATIONSPRODUKTEN**
COSMETIC PRODUCTS BASED ON URETHANE(METH)ACRYLATES CONTAINING SILOXANE GROUPS AND THEIR FREE RADICAL POLYMERISATION PRODUCTS
PRODUITS COSMETIQUES EN BASE DE URETHAN(METH)ACRYLATES RADICALEMENT POLYMERISABLES ET CONTENANT DES GROUPES SILOXANES AINSI QUE POLYMERE A BASE DE CES ACRYLATES

(30) Priorität: 26.08.1998 DE 19838852; 20.05.1999 DE 19923276
(43) Veröffentlichungstag der Anmeldung: 25.07.2001
(73) Patentinhaber: BASF Aktiengesellschaft, 67056 Ludwigshafen (DE)
(72) Erfinder: NGUYEN KIM, Son, D-69502 Hemsbach (DE); SANNER, Axel, D-67227 Frankenthal (DE); SCHEHLMANN, Volker, D-67105 Schifferstadt (DE)
(74) Vertreter: Kinzebach, Werner
(86) Internationale Anmeldenummer: PCT/EP1999/006234
(87) Internationale Veröffentlichungsnummer: WO 2000/012588

(56) Entgegenhaltungen:
- EP-A- 0 274 699
- EP-A- 0 408 311
- WO-A-97/38035

## Beschreibung

Die vorliegende Erfindung betrifft radikalisch polymerisierbare, siloxangruppenhaltige Urethan(meth)acrylate, wasserlösliche oder wasserdispergierbare Polymere, die diese einpolymerisiert enthalten, die Verwendung dieser Polymere sowie kosmetische Mittel, die diese Polymere enthalten.

In der Kosmetik werden Polymere mit filmbildenden Eigenschaften zur Festigung, Strukturverbesserung und Formgebung der Haare verwendet. Diese Haarbehandlungsmittel enthalten im Allgemeinen eine Lösung des Filmbildners in einem Alkohol oder einem Gemisch aus Alkohol und Wasser.

Haarfestigungsmittel werden im Allgemeinen in Form von wässrig-alkoholischen Lösungen auf die Haare aufgesprüht. Nach dem Verdampfen des Lösungsmittels werden die Haare an den gegenseitigen Berührungspunkten vom zurückbleibenden Polymer in der gewünschten Form gehalten. Die Polymere sollen einerseits so hydrophil sein, dass sie aus dem Haar ausgewaschen werden können, andererseits aber sollen sie hydrophob sein, damit die mit den Polymeren behandelten Haare auch bei hoher Luftfeuchtigkeit ihre Form behalten und nicht miteinander verkleben. Um eine möglichst effiziente Haarfestigerwirkung zu erzielen, ist es außerdem wünschenswert, Polymere einzusetzen, welche ein relativ hohes Molekulargewicht und eine relativ hohe Glastemperatur (mindestens 15°C) besitzen.

Bei der Formulierung von Haarfestigern ist außerdem zu berücksichtigen, dass aufgrund der Umweltbestimmungen zur Kontrolle der Emission flüchtiger organischer Verbindungen (VOC = volatile organic compounds) in die Atmosphäre eine Verringerung des Alkohol- und Treibmittelgehalts erforderlich ist.

Ein weiterer aktueller Anspruch an Haarbehandlungsmittel ist es, dem Haar ein natürliches Aussehen und Glanz zu verleihen, z. B. auch dann, wenn es sich um von Natur aus besonders kräftiges und/oder dunkles Haar handelt.

Es ist bekannt, Polysiloxane und Polysiloxanderivate, die nicht kovalent an ein Festigerpolymer gebunden sind, als weichmacherkomponente in Haarpflegemitteln einzusetzen. Da Siliconöle und Polysiloxanderivate mit Festigerpolymeren, die allgemein polare Gruppen enthalten, unverträglich sind, erfordert die Herstellung stabiler Formulierungen im Allgemeinen den Zusatz weiterer Hilfsstoffe. Häufig kommt es dennoch zu Entmischungen während der Lagerung oder nach Anwendung der Produkte auf den Haaren. Der Anwendungsbereich derartiger Formulierungen ist daher stark eingeschränkt. Um die nachteilige Entmischung zu verhindern, sind Versuche unternommen worden, Polysiloxangruppen kovalent an das Festigerpolymer zu binden.

Die WO-A-97/00664 beschreibt wässrige Nagellacke, die ein vernetztes Acrylharz auf Basis von difunktionellen Urethanacrylat-Oligomeren enthalten. Die mit diesen Harzen erhaltenen Verfilmungen sind weder wasserlöslich noch in Wasser redispergierbar. Eine Verwendung in der Haarkosmetik, insbesondere als Festigerpolymer, wird in diesem Dokument nicht beschrieben. Auch der Einsatz von siloxangruppenhaltigen Urethanacrylaten wird nicht beschrieben.

Die EP-A-408 311 beschreibt die Verwendung Copolymers, das a) ein ethylenisch ungesättigtes, hydrophiles Monomer, b) ein ethylenisch ungesättigtes Monomeren mit Polysiloxangruppen und c) ein ethylenisch ungesättigtes, hydrophobes Monomer eingebaut enthält, in Haarpflegeprodukten.

Die EP-A-412 704 beschreibt ein Haarpflegemittel auf Basis eines Pfropfcopolymers, welches monovalente Siloxan-Polymereinheiten an einem Rückgrat auf Basis eines vinylpolymers aufweist.

Die WO 93/03703 beschreibt eine Haarsprayzusammensetzung, umfassend: a) ein oberflächenaktives Mittel, b) ein ionisches Harz mit einem zahlenmittleren Molekulargewicht von mindestens 300 000 und c) einen flüssigen Träger. Dabei kann es sich bei dem ionischen Harz um die in der EP-A-412 704 beschriebenen Pfropfcopolymere handeln.

Die EP-A-362 860 beschreibt Alkohol-modifizierte Siliconesterderivate und kosmetische Zusammensetzungen, die diese enthalten.

Keine dieser Publikationen beschreibt Festigerpolymere auf Basis von α,β-ethylenisch ungesättigten Polyurethanen, die zusätzlich wenigstens eine Siloxangruppe aufweisen.

Es ist bekannt, Polyurethane mit filmbildenden Eigenschaften in der Kosmetik einzusetzen. So beschreiben die DE-A-42 25 045 und die WO 94/03515 die Verwendung von wasserlöslichen oder in wasser dispergierbaren, anionischen Polyurethanen als Haarfestiger. Die DE-A-42 41 118 beschreibt die Verwendung von kationischen Polyurethanen und Polyharnstoffen als Hilfsmittel in kosmetischen und pharmazeutischen Zubereitungen. Diese Polyurethane umfassen keine Polysiloxangruppen und können die Anforderungen an Haarfestigerpolymere nur teilweise erfüllen. So ist z. B. die Geschmeidigkeit des Haares verbesserungswürdig.

Die EP-A-492 657 beschreibt ein kosmetischen Mittel zur Verwendung in Haut- und Haarpflegeprodukten, welches ein lineares Polysiloxan-Polyoxyalkylen-Blockcopolymer enthält.

Die EP-A-0 389 386 beschreibt Blockcopolymere, die ein Polysiloxandiol, einen Blockcopolyester und ein Diisocyanat eingebaut enthalten. Sie eignen sich zur kontrollierten Freigabe aktiver Inhaltsstoffe.

Die EP-A-277 816 beschreibt Polydimethylsiloxane mit zwei Hydroxylgruppen an einem Kettenende und einer Trimethylsilylgruppe am anderen, der allgemeinen Formel 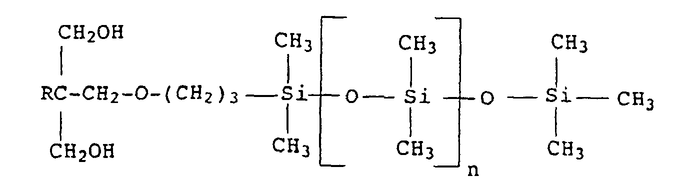 worin R für Wasserstoff, Methyl oder Ethyl steht und n für einen Wert von 0 bis 4 000 steht, sowie damit modifizierte Polyurethane. Die Herstellung dieser siloxanmodifizierten Polyurethane erfolgt durch Polykondensation der Polysiloxane der obigen Formel mit Polyurethanpräpolymeren, die zwei oder mehrere Isocyanatgruppen aufweisen. Radikalisch polymerisierbare siloxangruppenhaltige Polyurethane und Polymere, die diese einpolymerisiert enthalten, werden nicht beschrieben. Eine Anwendung der modifizierten Polyurethane in der Haarkosmetik wird auch nicht beschrieben.

Die EP-A-636 361 beschreibt eine kosmetische Zusammensetzung, welche in einem Träger mindestens einen Pseudolatex auf Basis eines Polykondensates umfasst, das mindestens eine Polysiloxaneinheit und mindestens eine Polyurethan- und/oder Polyharnstoffeinheit mit anionischen oder kationischen Gruppen enthält. Diese Polykondensate weisen keine radikalisch polymerisierbare, α,β-ethylenisch ungesättigte Doppelbindung auf und werden auch nicht zur Siliconmodifizierung anderer Polymere eingesetzt. Die Auswaschbarkeit dieser Filmbildner ist nicht zufriedenstellend. Zudem besitzen sie aufgrund eines hohen Siloxananteils auch nicht die für ein Haarpolymer erforderliche Festigungswirkung.

Die WO 97/25021 hat einen der EP-A-0 636 361 vergleichbaren Offenbarungsgehalt.

Die EP-A-751 162 beschreibt die Verwendung von Polykondensaten mit Polyurethan- und/oder Harnstoffeinheiten und einkondensierten Polysiloxaneinheiten oder aufgepfropften Polysiloxan-Seitenketten zur Herstellung von kosmetischen oder dermatologischen Zusammensetzungen. Die eingesetzten Komponenten entsprechend im Wesentlichen den in der EP-A-636 361 beschriebenen.

Die EP-A-0 705 594 beschreibt ein kosmetisches Mittel, das eine wäßrige Dispersion eines Filmbildnerpolymers und eine wasserlösliche oder wasserdispergierbare Siliconzusammensetzung enthält. Dabei kann es sich bei dem Filmbildnerpolymer um ein Polyurethan oder einen Polyharnstoff handeln.

Die DE-A-195 24 816 beschreibt hydroxylierte Siloxanblockcopolymere, die Siloxan- und Kohlenwasserstoffsegmente enthalten, welche über hydroxylierte Kohlenwasserstoffstrukturen verknüpft sind.

Die DE-A-195 41 326 und die WO 97/17386 beschreiben wasserlösliche oder wasserdispergierbare Polyurethane mit endständigen Säuregruppen. Zu ihrer Herstellung wird ein Polyurethanpräpolymer mit endständigen Isocyanatgruppen mit einer Aminosulfonsäure oder Aminocarbonsäure umgesetzt. Dabei können die Polyurethanpräpolymere auch Siloxanverbindungen mit zwei gegenüber Isocyanatgruppen reaktiven Gruppen einkondensiert enthalten, wobei die Publikation jedoch kein Beispiel für ein entsprechendes Polyurethanpräpolymer enthält.

Die DE-A-195 41 658 beschreibt wasserlösliche oder wasserdispergierbare Pfropfcopolymere aus einem Polyurethanpräpolymer mit endständigen Isocyanatgruppen und einem Aminogruppen enthaltenden Protein, wobei das Präpolymer auch Siloxangruppen eingebaut enthalten kann.

Radikalisch polymerisierbare, siloxangruppenhaltige Polyurethane und Polymere, die diese einpolymerisiert enthalten, werden in den zuvor genannten Dokumenten nicht beschrieben.

Die EP-A-687 459 beschreibt Haarbehandlungsmittel auf Basis einer wässrigen Polymerdispersion, die durch radikalische Pfropfcopolymerisation eines monoethylenisch ungesättigten Siloxanmakromonomers und wenigstens eines Polymers erhältlich ist, wobei es sich um Polyester oder Polyesteramide handeln kann. Radikalisch ungesättigte, siloxangruppenhaltige Polyurethane sind nicht beschrieben. Auch eine Umsetzung der monoethylenisch ungesättigten Siloxanmakromere mit weiteren, α,β-ethylenisch ungesättigten Komponenten ist nicht beschrieben.

Die EP-A-687 462 und die US-A-5,650,159 besitzen einen der EP 687 459 vergleichbaren Offenbarungsgehalt, wobei es sich bei den Polymeren, die mit den Siloxanmakromonomeren pfropfcopolymerisiert werden, um Polyurethane und/oder Polyharnstoffe handeln kann.

Die WO 95/00108 beschreibt flüssige Haarbehandlungsmittel auf Basis von Pfropfcopolymeren aus einem Vinylpolymer-Rückgrat und darauf aufgepfropften siliconhaltigen Macromeren. Das Vinylcopolymer-Rückgrat besteht dabei aus einem hydrophilen carbonsäurehaltigen Monomer und gegebenenfalls einem lipophilen Monomer. Siliconhaltige Macromere, die Diisocyanate eingebaut enthalten, sind in diesem Dokument nicht beschrieben.

Die US-A-5,162,472 beschreibt siliconhaltige Polymere, erhältlich durch radikalische Polymerisation eines vinylsiliconhaltigen Monomers, wobei es sich um (Meth)acrylsäureester von Siloxanpolyolen oder um vinylsiliconurethane auf Basis von Benzol-1-(1-isocyanato-1-methylethyl)-3-(1-(methyl)ethinyl) handeln kann. Radikalisch polymerisierbare siloxangruppenhaltige Polymere auf Basis von Diisocyanaten sind nicht beschrieben.

EP-A-0274699 beschreibt ein Verfahren zur Herstellung polymerisierbarer Copolymere auf Basis von (Meth)Acrylat-Urethan-Oligomeren und funktionalisierten Polysiloxanen. Eine Verwendung der hergestellten Copolymere im kosmetischen Bereich wird nicht erwähnt.

WO97/38035 beschreibt flüssige härtbare Zusammensetzungen, die eine ethylenisch ungesättigte Polydimethylsiloxan-Verbindung mit mindestens zwei Urethan-Bindungen in einem Molekül umfasst. Eine Verwendung im kosmetischen Bereich wird nicht erwähnt.

Der vorliegenden Erfindung liegt die Aufgabe zugrunde, neue, radikalisch polymerisierbare, siloxangruppenhaltige Urethan (meth) acrylate zur Verfügung zu stellen. Diese sollen sich zur Herstellung von siloxanmodifizierten Polymeren durch radikalische Polymerisation eignen. Vorzugsweise sollen die dabei resultierenden siloxanmodifizierten Polymere als Haarbehandlungsmittel geeignet sein. Insbesondere sollen diese Haarbehandlungsmittel einerseits als Haarfestiger brauchbar sein, andererseits aber auch eine gute Auswaschbarkeit (Redispergierbarkeit) besitzen.

Überraschenderweise wurde nun gefunden, dass die Aufgabe durch radikalisch polymerisierbare, siloxangruppenhaltige Urethan(meth)acrylate gelöst wird, die wenigstens eine Verbindung mit mindestens einem aktiven Wasserstoffatom und mindestens einer radikalisch polymerisierbaren, α,β-ethylenisch ungesättigten Doppelbindung, wenigstens ein Diisocyanat, wenigstens eine Verbindung mit 2 aktiven Wasserstoffatomen pro Molekül und wenigstens eine Verbindung mit wenigstens einer Siloxangruppe pro Molekül eingebaut enthalten.

Gegenstand der vorliegenden Erfindung ist daher ein Haarbehandlungsmittel, das wenigstens ein wasserlösliches oder wasserdispergierbares Polymer sowie übliche kosmetische Hilfsstoffe enthält, wobei das Polymer wenigstens ein Urethan(meth)acrylat und wenigstens ein radikalisch polymerisierbares, α,β-ethylenisch ungesättigtes Monomer M) einpolymerisiert enthält, wobei das Urethan(meth)acrylat ausgewählt ist unter radikalisch polymerisierbaren, siloxangruppenhaltigen Urethan(meth)acrylaten, die
a) wenigstens eine Verbindung, die mindestens ein aktives Wasserstoffatom und mindestens eine radikalisch polymerisierbare, α,β-ethylenisch ungesättigte Doppelbindung pro Molekül enthält,
b) wenigstens ein Diisocyanat,
c) wenigstens eine Verbindung, die zwei aktive Wasserstoffatome pro Molekül enthält,
d) wenigstens eine Verbindung, die mindestens ein aktives Wasserstoffatom und mindestens eine Siloxangruppe pro Molekül enthält,
eingebaut enthalten, und den Salzen davon.

Im Rahmen der vorliegenden Erfindung umfasst der Ausdruck "Urethan(meth)acrylate" auch Verbindungen, die Harnstoffgruppen statt oder zusätzlich zu den Urethangruppen aufweisen. Harnstoffgruppen resultieren bei der Umsetzung einer Isocyanatgruppe mit einer primären oder sekundären Aminogruppe. Zur Herstellung von Urethan(meth)acrylaten mit Harnstoffgruppen können Komponenten mit aktiven Wasserstoffatomen a), c) und/oder d) sowie gegebenenfalls e) und/oder f) eingesetzt werden, die wenigstens eine Verbindung mit mindestens einer primären und/oder sekundären Aminogruppe enthalten.

Im Rahmen der vorliegenden Erfindung umfasst der Ausdruck C₁- bis C₃₀-'Alkyl' geradkettige und verzweigte Alkylgruppen. Geeignete kurzkettige Alkylgruppen sind z. B. geradkettige oder verzweigte C₁-C₈-Alkyl-, bevorzugt C₁-C₆-Alkyl- und besonders bevorzugt C₁-C₄-Alkylgruppen. Dazu zählen insbesondere Methyl, Ethyl, Propyl, Isopropyl, n-Butyl, 2-Butyl, sec.-Butyl, tert.-Butyl, n-Pentyl, 2-Pentyl, 2-Methylbutyl, 3-Methylbutyl, 1,2-Dimethylpropyl, 1,1-Dimethylpropyl, 2,2-Dimethylpropyl, 1-Ethylpropyl, n-Hexyl, 2-Hexyl, 2-Methylpentyl, 3-Methylpentyl, 4-Methylpentyl, 1,2-Dimethylbutyl, 1,3-Dimethylbutyl, 2,3-Dimethylbutyl, 1,1-Dimethylbutyl, 2,2-Dimethylbutyl, 3,3-Dimethylbutyl, 1,1,2-Trimethylpropyl, 1,2,2-Trimethylpropyl, 1-Ethylbutyl, 2-Ethylbutyl, 1-Ethyl-2-methylpropyl, n-Heptyl, 2-Heptyl, 3-Heptyl, 2-Ethylpentyl, 1-Propylbutyl, Octyl etc.

Geeignete längerkettige C₈- bis C₃₀-Alkyl- bzw. C₈- bis C₃₀-Alkylengruppen sind geradkettige und verzweigte Alkyl- bzw. Alkylengruppen. Bevorzugt handelt es sich dabei um überwiegend lineare Alkylreste, wie sie auch in natürlichen oder synthetischen Fettsäuren und Fettalkoholen sowie Oxoalkoholen vorkommen, die gegebenenfalls zusätzlich einfach, zweifach oder mehrfach ungesättigt sein können. Dazu zählen z. B. n-Hexyl(en), n-Heptyl(en), n-Octyl(en), n-Nonyl(en), n-Decyl(en), n-Undecyl(en), n-Dodecyl(en), n-Tridecyl(en), n-Tetradecyl(en), n-Pentadecyl(en), n-Hexadecyl(en), n-Heptadecyl(en), n-Octadecyl(en), n-Nonadecyl(en) etc.

Bei der C₅- bis C₈-Cycloalkylgruppe handelt es sich z. B. um Cyclopentyl, Cyclohexyl, Cycloheptyl oder Cyclooctyl.

Im Folgenden werden Verbindungen, die sich von Acrylsäure und Methacrylsäure ableiten können teilweise verkürzt durch Einfügen der Silbe "(meth)" in die von der Acrylsäure abgeleitete Verbindung bezeichnet.

### Komponente a)

Geeignete Verbindungen a) sind z.B. die üblichen, dem Fachmann bekannten Vinylverbindungen, die zusätzlich wenigstens eine gegenüber Isocyanatgruppen reaktive Gruppe aufweisen, die vorzugsweise ausgewählt ist unter Hydroxylgruppen sowie primären und sekundären Aminogruppen. Dazu zählen z. B. die Ester α,β-ethylenisch ungesättigter Mono- und Dicarbonsäuren mit mindestens zweiwertigen Alkoholen. Als α,β-ethylenisch ungesättigte Mono- und/oder Dicarbonsäuren können z.B. Acrylsäure, Methacrylsäure, Fumarsäure, Maleinsäure, Crotonsäure, Itaconsäure etc. und Gemische davon eingesetzt werden. Geeignete Alkohole sind übliche Diole, Triole und Polyole, z. B. 1,2-Ethandiol, 1,3-Propandiol, 1,4-Butandiol, 1,5-Pentandiol, 1,6-Hexandiol, 1,10-Decandiol, Diethylenglykol, 2,2,4-Trimethylpentandiol-1,5, 2,2-Dimethylpropandiol-1,3, 1,4-Dimethylolcyclohexan, 1,6-Dimethylolcyclohexan, Glycerin, Trimethylolpropan, Erythrit, Pentaerythrit, Sorbit etc. Bei den Verbinden a) handelt es sich dann z. B. um Hydroxymethyl(meth)acrylat, Hydroxyethylethacrylat, 2-Hydroxyethyl(meth)acrylat, 2-Hydroxypropyl(meth)acrylat, 3-Hydroxypropyl(meth)acrylat, 3-Hydroxybutyl(meth)acrylat, 4-Hydroxybutyl(meth)acrylat, 6-Hydroxyhexyl(meth)acrylat, 3-Hydroxy-2-ethylhexyl(meth)acrylat sowie um Di(meth)acrylsäureester des 1,1,1-Trimethylolpropans oder des Glycerins.

Geeignete Monomere a) sind weiterhin die Ester und Amide der zuvor genannten α,β-ethylenisch ungesättigten Mono- und Dicarbonsäuren mit C₂- bis C₁₂-Aminoalkoholen, die eine primäre oder sekundäre Aminogruppe aufweisen. Dazu zählen Aminoalkylacrylate und Aminoalkylmethacrylate und deren N-Monoalkylderivate, die z. B. einen N-C₁- bis C₈-Monoalkylrest tragen, wie Aminomethyl(meth)acrylat, Aminoethyl(meth)acrylat, N-Methylaminomethyl(meth)acrylat, N-Ethylaminomethyl(meth)acrylat, N-Ethylaminoethyl(meth)acrylat, N-(n-propyl)aminomethyl(meth)acrylat, N-Isopropylaminomethyl(meth)acrylat und bevorzugt tert.-Butylaminoethylacrylat und tert.-Butylaminoethylmethacrylat. Dazu zählen weiterhin N-(Hydroxy-C₁- bis C₁₂-alkyl)(meth)acrylamide, wie N-Hydroxymethyl(meth)acrylamid, N-Hydroxyethyl(meth)acrylamid etc.

Geeignete Monomere a) sind auch die Amide der zuvor genannten α,β-ethylenisch ungesättigten Mono- und Dicarbonsäuren mit Di- und Polyaminen, die mindestens zwei primäre oder zwei sekundäre oder eine primäre und eine sekundäre Aminogruppe(n) aufweisen. Dazu zählen z. B. die entsprechenden Amide der Acrylsäure und Methacrylsäure, wie Aminomethyl(meth)acrylamid, Aminoethyl(meth)acrylamid, Aminopropyl (meth) acrylamid, Amino-n-butyl(meth)acrylamid, Methylaminoethyl (meth) acrylamid, Ethylaminoethyl(meth)acrylamid, Methylaminopropyl (meth) acrylamid, Ethylaminopropyl(meth)acrylamid, Methylamino-n-butyl(meth)acrylamid etc.

Geeignete Monomere a) sind auch die Reaktionsprodukte von Epoxidverbindungen, die mindestens eine Epoxidgruppe aufweisen, mit den zuvor genannten α,β-ethylenisch ungesättigten Mono- und/oder Dicarbonsäuren und deren Anhydriden. geeignete Epoxidverbindungen sind z. B. Glycidylether, wie Bisphenol-A-diglycidylether, Resorcindiglycidylether, 1,3-Propandioldiglycidylether, 1,4-Butandioldiglycidylether, 1,5 Pentandioldiglycidylether, 1,6-Hexandioldiglycidylether etc.

### Komponente b)

Bei der Komponente b) handelt es sich um übliche aliphatische, cycloaliphatische und/oder aromatische Diisocyanate, wie Tetramethylendiisocyanat, Hexamethylendiisocyanat, Methylendiphenyldiisocyanat, 2,4- und 2,6-Toluylendiisocyanat und deren Isomerengemische, o- und m-Xylylendiisocyanat, 1,5-Naphthylendiisocyanat, 1,4-Cyclohexylendiisocyanat, Dicyclohexylmethandiisocyanat und Mischungen davon. Bevorzugt handelt es sich bei der Komponente b) um Hexamethylendiisocyanat, Isophorondiisocyanat, o- und m-Xylylendiisocyanat, Dicyclohexylmethandiisocyanat und Mischungen davon. Gewünschtenfalls können bis zu 3 Mol-% der genannten Verbindungen durch Triisocyanate ersetzt sein.

### Komponente c)

Geeignete Verbindungen der Komponente c) sind z. B. Diole, Diamine, Aminoalkohole, und Mischungen davon. Das Molekulargewicht dieser Verbindungen liegt vorzugsweise in einem Bereich von etwa 56 bis 280. Gewünschtenfalls können bis zu 3 Mol-% der genannten Verbindungen durch Triole oder Triamine ersetzt sein.

Geeignete Diole c) sind z. B. Ethylenglykol, Propylenglykol, Butylenglykol, Neopentylglykol, Cyclohexandimethylol, Di-, Tri-, Tetra-, Penta- oder Hexaethylenglykol und Mischungen davon. Bevorzugt werden Neopentylglykol und/oder Cyclohexandimethylol eingesetzt.

Geeignete Aminoalkohole c) sind z. B. 2-Aminoethanol, 2-(N-Methylamino)ethanol, 3-Aminopropanol, 4-Aminobutanol, 1-Ethylaminobutan-2-ol, 2-Amino-2-methyl-1-propanol, 4-Methyl-4-aminopentan-2-ol etc.

Geeignete Diamine c) sind z. B. Ethylendiamin, Propylendiamin, 1,4-Diaminobutan, 1,5-Diaminopentan und 1,6-Diaminohexan.

Bevorzugte Verbindungen der Komponente c) sind Polymerisate mit einem zahlenmittleren Molekulargewicht im Bereich von etwa 300 bis 5 000, bevorzugt etwa 400 bis 4 000, insbesondere 500 bis 3 000. Dazu zählen z. B. Polyesterdiole, Polyetherole, α,ω-Diaminopolyether und Mischungen davon. Vorzugsweise werden ethergruppenhaltige Polymerisate eingesetzt.

Bei den Polyetherolen c) handelt es sich vorzugsweise um Polyalkylenglykole, z.B. Polyethylenglykole, Polypropylenglykole, Polytetrahydrofurane etc., Blockcopolymerisate aus Ethylenoxid und Propylenoxid oder Blockcopolymerisate aus Ethylenoxid, Propylenoxid und Butylenoxid, die die Alkylenoxideinheiten statistisch verteilt oder in Form von Blöcken einpolymerisiert enthalten.

Geeignete α,ω-Diaminopolyether c) sind z. B. durch Aminierung von Polyalkylenoxiden mit Ammoniak herstellbar.

Geeignete Polytetrahydrofurane c) können durch kationische Polymerisation von Tetrahydrofuran in Gegenwart von sauren Katalysatoren, wie z. B. Schwefelsäure oder Fluoroschwefelsäure, hergestellt werden. Derartige Herstellungsverfahren sind dem Fachmann bekannt.

Brauchbare Polyesterdiole c) weisen vorzugsweise ein zahlenmittleres Molekulargewicht im Bereich von etwa 400 bis 5 000, bevorzugt 500 bis 3 000, insbesondere 600 bis 2 000, auf.

Als Polyesterdiole kommen alle diejenigen in Betracht, die üblicherweise zur Herstellung von Polyurethanen eingesetzt werden, insbesondere solche auf Basis aromatischer Dicarbonsäuren, wie Terephthalsäure, Isophthalsäure, Phthalsäure, Na- oder K-Sulfoisophthalsäure etc., aliphatischer Dicarbonsäuren, wie Adipinsäure oder Bernsteinsäure etc., und cycloaliphatischer Dicarbonsäuren, wie 1,2-, 1,3- oder 1,4-Cyclohexandicarbonsäure. Als Diole kommen insbesondere aliphatische Diole in Betracht, wie Ethylenglykol, Propylenglykol, 1,6-Hexandiol, Neopentylglykol, Diethylenglykol, Polyethylenglykole, Polypropylenglykole, 1,4-Dimethylolcyclohexan, sowie Poly(meth)acrylatdiole der Formel 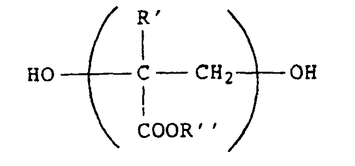 worin R' für H oder CH₃ steht und R" für C₁-C₁₈-Alkyl (insbesondere C₁-C₁₂- oder C₁-C₈-Alkyl) steht, die eine Molmasse von bis zu etwa 3000 aufweisen. Derartige Diole sind auf übliche Weise herstellbar und im Handel erhältlich (Tegomer®-Typen MD, BD und OD der Fa. Goldschmidt).

Bevorzugt sind Polyesterdiole auf Basis von aromatischen und aliphatischen Dicarbonsäuren und aliphatischen Diolen, insbesondere solche, bei denen die aromatische Dicarbonsäure 10 bis 95 Mol-%, insbesondere 40 bis 90 Mol-% und bevorzugt 50 bis 85 Mol-%, des gesamten Dicarbonsäureanteils (Rest aliphatische Dicarbonsäuren) ausmacht.

Besonders bevorzugte Polyesterdiole sind die Umsetzungsprodukte aus Phthalsäure/Diethylenglykol, Isophthalsäure/1,4-Butandiol, Isophthalsäure/Adipinsäure/1,6-Hexandiol, 5-NaSO₃-Isophthalsäure/ Phthalsäure/Adipinsäure/1,6-Hexandiol, Adipinsäure/Ethylenglykol, Isophthalsäure/Adipinsäure/Neopentylglykol, Isophthalsäure/Adipinsäure/Neopentylglykol/Diethylenglykol/Dimethylolcyclohexan und 5-NaSO₃-Isophthalsäure/Isophthalsäure/Adipinsäure/Neopentylglykol/ Diethylenglykol/Dimethylolcyclohexan.

Die Verbindungen der Komponente c) können einzeln oder als Mischungen eingesetzt werden.

### Komponente d)

Bevorzugt ist die Komponente d) ausgewählt unter:
- Polysiloxanen der allgemeinen Formel I.1 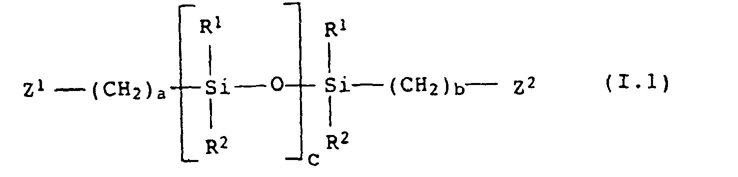 worin
   - a und b: unabhängig voneinander für 2 bis 8 stehen,
   - c: für 3 bis 100 steht,
   - R¹ und R²: unabhängig voneinander für C₁- bis C₈-Alkyl, Benzyl oder Phenyl stehen,
   - Z¹ und Z²: unabhängig voneinander für OH, NHR³ oder einen Rest der Formel II

   -O-(CH₂CH₂O)ᵥ(CH₂CH(CH₃)O)_{w}-H (II)

   stehen, wobei
   in der Formel II die Reihenfolge der Alkylenoxideinheiten beliebig ist und
   v und w unabhängig voneinander für eine ganze Zahl von 0 bis 200 stehen, wobei die Summe aus v und w > 0 ist,
   R³ für Wasserstoff, C₁- bis C₈-Alkyl oder C₅- bis C₈-Cycloalkyl steht;
- Polysiloxanen der allgemeinen Formel 1.2 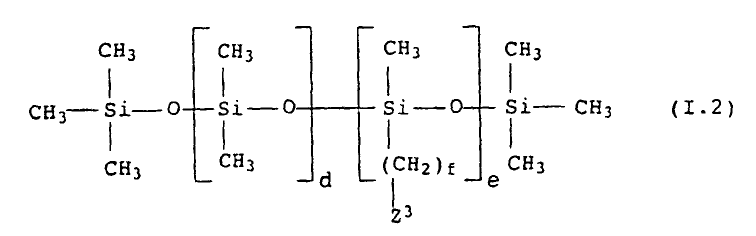 worin
   die Reihenfolge der Siloxaneinheiten beliebig ist,
   - d und e: unabhängig voneinander für 0 bis 100 stehen, wobei die Summe aus d und e mindestens 2 ist,
   - f: für eine ganze Zahl von 2 bis 8 steht,
   - Z³: für OH, NHR³ oder einen Rest der Formel II steht,
   wobei R³ für Wasserstoff, C₁- bis C₈-Alkyl, C₅- bis C₈₋Cycloalkyl oder einen Rest der Formel -(CH₂)ᵤ-NH₂ steht, wobei u für eine ganze Zahl von 1 bis 10, bevorzugt 2 bis 6, steht,
- Polysiloxanen mit sich wiederholenden Einheiten der allgemeinen Formel I.3 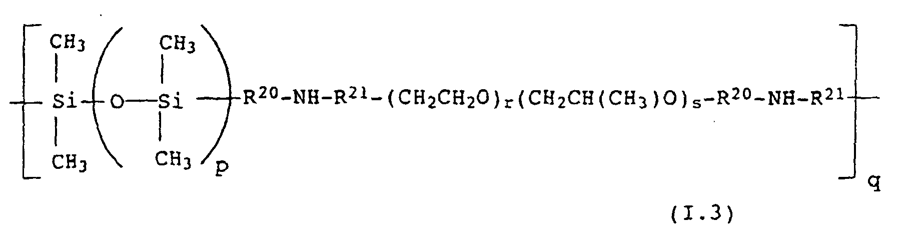 worin
   - p: für eine ganze Zahl von 0 bis 100 steht,
   - q: für eine ganze Zahl von 1 bis 8 steht,
   - R²⁰ und R²¹: unabhängig voneinander für C₁- bis C₈-Alkylen stehen,
   - die: Reihenfolge der Alkylenoxideinheiten beliebig ist und
   - r und s: unabhängig voneinander für eine ganze Zahl von 0 bis 200 stehen, wobei die Summe aus r und s > 0 ist,
- Polysiloxanen der allgemeinen Formel I.4 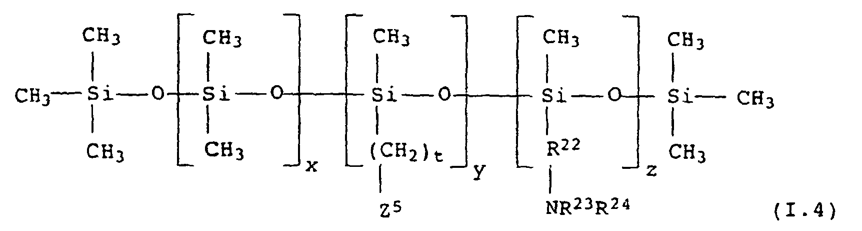 wor in
   - R²²: für einen C₁- bis C₈-Alkylenrest steht,
   - R²³ und R²⁴: unabhängig voneinander für Wasserstoff, C₁- bis C₈-Alkyl oder C₅- bis C₈-Cycloalkyl stehen,
   die Reihenfolge der Siloxaneinheiten beliebig ist,
   - x, y und z: unabhängig voneinander für 0 bis 100 stehen, wobei die Summe aus x, y und z mindestens 3 ist,
   - t: für eine ganze Zahl von 2 bis 8 steht,
   - Z⁵: für einen Rest der Formel VII

   -(OCH₂CH₂)ᵢ(OCH₂CH(CH₃))ⱼ-R²⁵ (VII)

   steht, worin
   die Reihenfolge der Alkylenoxideinheiten beliebig ist und i und j unabhängig voneinander für eine ganze Zahl von 0 bis 200 stehen, wobei die Summe aus i und j > O ist,

   - R²⁵: für Wasserstoff oder einen C₁- bis C₈-Alkylrest steht.
und Mischungen davon.

Nach einer geeigneten Ausführungsform weisen die Polysiloxane d) der allgemeinen Formel I.1 keine Alkylenoxidreste der allgemeinen Formel II auf. Diese Polysiloxane d) weisen dann vorzugsweise ein zahlenmittleres Molekulargewicht im Bereich von etwa 300 bis 5 000, bevorzugt 400 bis 3 000 auf.

Geeignete Polysiloxane d), die keine Alkylenoxidreste aufweisen sind z.B. die Tegomer® -Marken der Fa. Goldschmidt.

Nach einer weiteren geeigneten Ausführungsform handelt es sich bei den Polysiloxanen d) um Silicon-poly(alkylenoxid)-Copolymere der Formel I.1, wobei wenigstens einer oder beide Reste Z¹ und/oder Z² für einen Rest der allgemeinen Formel II stehen.

Vorzugsweise ist in der Formel II die Summe aus v und w so gewählt, daß das Molekulargewicht der Polysiloxane d) dann in einem Bereich von etwa 300 bis 30000 liegt.

Bevorzugt liegt die Gesamtzahl der Alkylenoxideinheiten der Polysiloxane d), das heißt die Summe aus v und w in der Formel II dann in einem Bereich von etwa 3 bis 200, bevorzugt 5 bis 180.

Nach einer weiteren geeigneten Ausführungsform handelt es sich bei den polysiloxanen d) um Silicon-poly(alkylenoxid)-Copolymere der Formel I.2, die wenigstens einen Rest Z³ der allgemeinen Formel II aufweisen.

Vorzugsweise ist dann in der Formel II die Summe aus v und w wiederum so gewählt, daß das Molekulargewicht der Polysiloxane d) dann in einem Bereich von etwa 300 bis 30000 liegt. Bevorzugt liegt die Gesamtzahl der Alkylenoxideinheiten der Polysiloxane d), das heißt die Summe aus v und w in der Formel II dann ebenfalls in einem Bereich von etwa 3 bis 200, bevorzugt 5 bis 180.

Geeignete Silicon-poly(alkylenoxid)-Copolymere d), die unter dem internationalen Freinamen Dimethicon bekannt sind, sind die Tegopren® -Marken der Fa. Goldschmidt, Belsil® 6031 der Fa. Wacker und Silvet® L der Fa. Witco.

Nach einer bevorzugten Ausführungsform handelt es sich bei den Polysiloxanen d) um Silicon-poly(alkylenoxid)-Copolymere der Formel I.2. die wenigstens einen Rest Z³ aufweisen, worin Z³ für NHR³ steht und R³ für Wasserstoff oder einen Rest der Formel -(CH₂)ᵤ-NH₂ steht. Vorzugsweise steht u für eine ganze Zahl von 1 bis 10, bevorzugt 2 bis 6. Dazu zählen z.B. die MAN- und MAR-Marken der Fa. Hüls sowie die Finish-Marken der Fa. Wacker, z.B. Finish WT 1270.

Bevorzugt umfassen die Polysiloxane d) wenigstens eine Verbindung der allgemeinen Formel I.3. Bevorzugt stehen in der Formel I.3 R²⁰ und R²¹ unabhängig voneinander für einen C₂- bis C₄-Alkylenrest. Insbesondere stehen R²⁰ und R²¹ unabhängig voneinander für einen C₂- bis C₃-Alkylenrest.

Vorzugsweise liegt das Molekulargewicht der Verbindung der Formel I.3 in einem Bereich von etwa 300 bis 100 000 liegt.

Vorzugsweise steht in der Formel I.3 p für eine ganze Zahl von 1 bis 20, wie z. B. 2 bis 10.

Bevorzugt liegt die Gesamtzahl der Alkylenoxideinheiten der Verbindung der Formel I.3, d. h. die Summe aus r und s, in einem Bereich von etwa 3 bis 200, bevorzugt 5 bis 180.

Bevorzugt sind die Endgruppen der Polysiloxane mit wiederholungseinheiten der allgemeinen Formel 1.3 ausgewählt unter (CH₃)₃SiO, H, C₁- bis C₈-Alkyl und Mischungen davon.

Aminogruppenhaltigen Verbindungen mit Wiederholungseinheiten der allgemeinen Formel 1.3 weisen bevorzugt eine Aminzahl in einem Bereich von etwa 2 bis 50, insbesondere 3 bis 20 auf.

Geeignete alkoxylierte Siloxanamine der Formel I.3 sind z. B. in der WO-A-97/32917 beschrieben, auf die hier in vollem Umfang Bezug genommen wird. Kommerziell erhältliche Verbindungen sind z. B. die Silsoft® -Marken der Fa. Witco, z. B. Silsoft® A-843.

Bevorzugt steht in der Formel I.4 der Rest R²² für einen C₂- bis C₄-Alkylenrest.

Bevorzugt stehen in der Formel I.4 R²³ und R²⁴ unabhängig voneinander für Wasserstoff oder C₁- bis C₄-Alkyl.

Vorzugsweise wird die Summe aus x, y und z so gewählt, dass das Molekulargewicht der Verbindung der Formel I.4 in einem Bereich von etwa 300 bis 100 000, bevorzugt 500 bis 50 000, liegt.

Bevorzugt liegt die Gesamtzahl der Alkylenoxideinheiten des Restes der Formel VII, d. h. die Summe aus i und j, in einem Bereich von etwa 3 bis 200, bevorzugt 5 bis 80.

Bevorzugt steht in der Formel VII der Rest R²⁵ für Wasserstoff oder C₁- bis C₄-Alkyl.

Eine geeignete Verbindung der Formel I.4 ist z. B. Silsoft® A-858 der Fa. Witco.

Geeignete Polysiloxane d) sind auch die in der EP-A-277 816 beschriebenen Polydimethylsiloxane.

Vorzugsweise enthalten die erfindungsgemäß eingesetzten Urethan(meth)acrylate zusätzlich wenigstens eine Komponente eingebaut, die ausgewählt ist unter
e) Verbindungen, die zwei oder mehrere aktive Wasserstoffatome und mindestens eine ionogene und/oder ionische Gruppe pro Molekül enthalten,
f) einwertigen Alkoholen, Aminen mit einer primären oder sekundären Aminogruppe, aliphatischen, cycloaliphatischen oder aromatischen Monoisocyanaten und Mischungen davon,
g) α,β-ethylenisch ungesättigten Verbindungen, die zusätzlich wenigstens eine Isocyanatgruppe pro Molekül enthalten,
und Mischungen davon.

### Komponente e)

Die erfindungsgemäß eingesetzten Urethan(meth)acrylate können zusätzlich wenigstens eine Komponente e) einpolymerisiert enthalten, die mindestens eine ionogene und/oder ionische Gruppe pro Molekül enthält. Vorzugsweise handelt es sich dabei um Carboxylatgruppen und/oder Sulfonatgruppen oder um stickstoffhaltige Gruppen.

Geeignete Diamine und/oder Diole e) mit ionogenen oder ionischen Gruppen sind z. B. Dimethylolpropansäure und Verbindungen der Formel 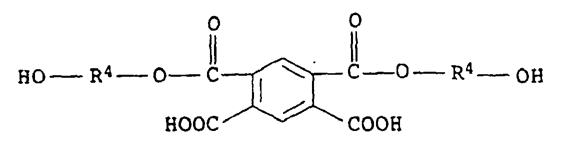 und/oder 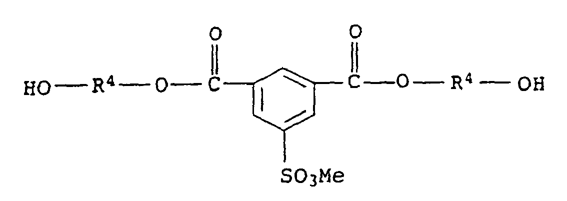 worin R⁴ jeweils für eine C₂-C₁₈-Alkylengruppe steht und Me für Na oder K steht.

Als Komponente e) brauchbar sind auch Verbindungen der Formel

H₂N(CH₂)ₙ-NH-(CH₂)ₘ-COO-M⁺

und/oder

H₂N(CH₂)ₙ-NH-(CH₂)ₘ-SO₃- M⁺

worin m und n unabhängig voneinander für eine ganze Zahl von 1 bis 8, insbesondere 1 bis 6, stehen und M für Li, Na oder K steht.

Wenn man als Komponente e) Verbindungen mit stickstoffhaltigen Gruppen verwendet, erhält man kationische Urethan(meth)acrylate. Brauchbare Komponenten e) sind z. B. Verbindungen der allgemeinen Formeln 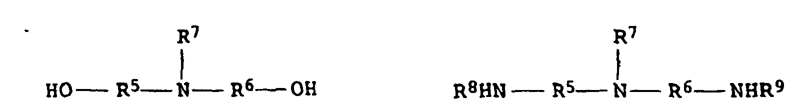 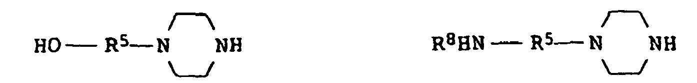 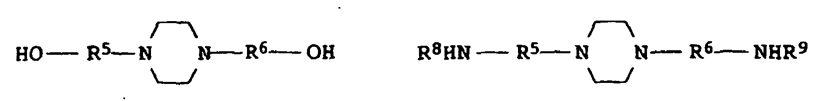 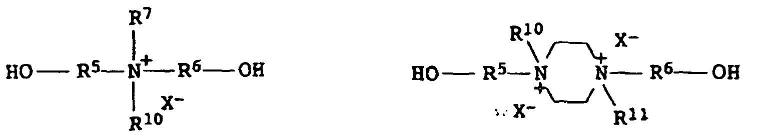 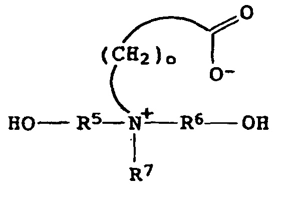 worin
- R⁵ und R⁶, die gleich oder verschieden sein können, für C₂-C₈-alkylen stehen,
- R⁷, R¹⁰ und R¹¹, die gleich oder verschieden sein können, für C₁-C₆-Alkyl, Phenyl oder Phenyl-C₁-C₄-alkyl stehen, R⁸ und R⁹, die gleich oder verschieden sein können, für H oder C₁-C₆-Alkyl stehen,
   o für 1, 2 oder 3 steht,
   X^{⊖} für Chlorid, Bromid, Jodid, C₁-C₆-Alkylsulfat oder SO₄²⁻/₂ steht.

### Komponente f)

Die erfindungsgemäß eingesetzten Urethan(meth)acrylate können eine Komponente f) eingebaut enthalten, die ausgewählt ist unter einwertigen Alkoholen, Aminen mit einer primären oder sekundären Aminogruppe, aliphatischen, cycloaliphatischen oder aromatischen Monoisocyanaten und Mischungen davon. Bevorzugte Verbindungen f) sind allgemein Verbindungen der Formel R¹²-Z⁴, worin R¹² für einen C₂₋bis C₃₀-Alkylrest steht und Z⁴ die in der Formel I.1 für Z¹ und Z² angegebenen Bedeutungen besitzt oder für NCO steht.

Geeignete einwertige Alkohole f) weisen einen geradkettigen oder verzweigten Alkylrest mit 2 bis 30 Kohlenstoffatomen, bevorzugt 8 bis 22 Kohlenstoffatomen, auf, der gegebenenfalls zusätzlich einfach, zweifach oder mehrfach ungesättigt sein kann. Geeignete C₂- bis C₃₀-Alkylreste sind die zuvor genannten. Die Alkohole f) können einzeln oder als Gemische eingesetzt werden.

Bevorzugte einwertige Alkohole f) sind z.B. Ethanol, n-Propanol, Nonanol. Undecanol, Tridecanol, Tetradecanol, Pentadecanol, Hexadecanol, Heptadecanol, Octadecanol, etc. und Mischungen davon. Die Alkohole können dabei isomerenrein oder in Form von Isomerengemischen eingesetzt werden.

Bevorzugte hydroxylgruppenhaltige Verbindungen f) sind weiterhin die Alkoxilate der zuvor genannten C₂- bis C₃₀-Alkanole, der allgemeinen Formel III

R¹²-(CH₂CH₂O)_{g}(CH₂CH(CH₃)O)ₕ-H (III)

wobei in der Formel III die Reihenfolge der Alkylenoxideinheiten beliebig ist, g und h unabhängig voneinander für eine ganze Zahl von 0 bis 200 stehen, wobei die Summe aus g und h > 0 ist, und R¹² die zuvor angegebene Bedeutung besitzt.

Geeignete höhere primäre oder sekundäre Amine f) sind Amine und Amingemische, die einen oder zwei der zuvor genannten C₁- bis C₃₀-Alkylreste aufweisen. Diese können z.B. durch Umsetzung natürlicher oder synthetischer Fettsäuren oder Fettsäuregemische mit Ammoniak zu Nitrilen und anschließende Hydrierung erhalten werden. Dazu zählen z.B. Alkylamine, die die zuvor bei den einwertigen Alkoholen f) genannten Alkylreste aufweisen, das heißt Ethyl- und die isomeren Propyl-, Butyl-, Pentyl-, Hexyl-, Heptyl-, Octyl-, Nonyl-, Decyl-, Undecyl-, Dodecyl-, Tridecyl-, Tetradecyl-, pentadecyl-, Hexadecyl-, Heptadecyl-, Octadecylamine etc. und Mischungen davon.

Geeignete Monoisocyanate f) sind z.B. C₁- bis C₃₀-Alkylisocyanate, die aus den zuvor genannten Aminen und Amingemischen durch Phosgenierung oder aus natürlichen oder synthetischen Fettsäuren und Fettsäuregemischen durch Hofmann-, Curtius- oder Lossen-Abbau erhältlich sind.

Geeignete cycloaliphatische Monoisocyanate f) sind z.B. Cyclohexylisocyanat, 2-, 3- und 4-Methylcyclohexylisocyanat, etc. und Mischungen davon.

Geeignete aromatische Monoisocyanate f) sind z.B. Phenylisocyanat, 2-, 3- und 4-Methylphenylisocyanat, etc. und Mischungen davon.

### Komponente g)

Bei der Komponente g) handelt es sich z. B. um Isocyanate der allgemeinen Formel IV 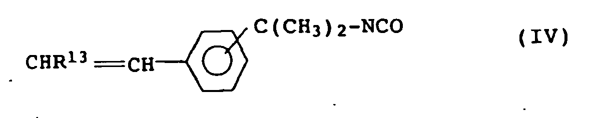 worin
die -C(CH₃)₂-NCO-Gruppe in o-, m- oder p-Stellung zur Vinylgruppe stehen kann und R¹³ für Wasserstoff oder C₁- bis C₈-Alkyl steht.

Bevorzugt steht in der Formel IV R¹³ für Wasserstoff, Methyl oder Ethyl.

Nach einer ersten möglichen Ausführungsform handelt es sich bei den erfindungsgemäß eingesetzten Urethan(meth)acrylaten um nichtionische Verbindungen. Diese enthalten dann keine der zuvor genannten Verbindungen e) eingebaut.

Nach einer zweiten möglichen Ausführungsform handelt es sich bei den erfindungsgemäß eingesetzten Urethan (meth) acrylaten um ionische Verbindungen. Diese weisen dann mindestens eine ionische oder ionogene Gruppe auf, d. h. sie enthalten mindestens eine der zuvor genannten Verbindungen der Komponente e) eingebaut. Wenn diese Verbindungen e) als ionogene bzw. ionische Gruppe wenigstens eine Carboxylatgruppe und/oder Sulfonatgruppe aufweiten, so resultieren anionische Urethan(meth)acrylate. Vorzugsweise wird zur Herstellung dieser anionischen Verbindungen Dimethylolpropansäure als Verbindung e) eingesetzt. Wenn es sich bei der Verbindung der Komponente e) um eine Verbindung mit einer stickstoffhaltigen Gruppe handelt, so resultieren kationische Urethan(meth)acrylate. Eine bevorzugte Verbindung e) zur Herstellung kationischer Urethan(meth)acrylate ist N-Methyldipropylentriamin.

Die Herstellung der erfindungsgemäß eingesetzten urethan(meth)acrylate erfolgt durch Umsetzung wenigstens eines Diisocyanates b) sowie gegebenenfalls einer oder mehrerer isocyanatgruppenhaltiger Verbindungen f) und/oder g) mit den gegenüber Isocyanatgruppen reaktiven Gruppen der übrigen Komponenten a), c), d) sowie gegebenenfalls e) und/oder f). Werden hydroxylgruppenhaltige Komponenten zur Umsetzung mit den isocyanatgruppenhaltigen Komponenten eingesetzt, so wird vorzugsweise die gesamte Isocyanatmenge mit den hydroxylgruppenhaltigen Komponenten zu einem isocyanatgruppenhaltigen Polyurethanpräpolymer umgesetzt. Diese Umsetzung erfolgt im Allgemeinen bei einer erhöhten Temperatur im Bereich von etwa 40 bis 150 °C, bevorzugt etwa 50 bis 100 °C. Die Reaktion kann ohne Lösungsmittel in der Schmelze oder in einem geeigneten Lösungsmittel oder Lösungsmittelgemisch erfolgen. Dazu zählen Ketone, wie Aceton und Methylethylketon. Bei der Umsetzung der isocyanatgruppenhaltigen Verbindungen der Komponenten b), f) und/oder g) mit den hydroxylgruppenhaltigen Verbindungen der Komponenten a), c), d) sowie e) und/oder f) wird das Verhältnis von NCO-Äquivalent der Verbindungen der Komponenten b), f) und/oder g) zu Äquivalent aktives Wasserstoffatom der übrigen Komponenten so gewählt, dass ein Polyurethanpräpolymer resultiert, welches noch freie Isocyanatgruppen aufweist. Im Allgemeinen beträgt das Verhältnis von NCO-Äquivalent zu Äquivalent aktives Wasserstoffatom der hydroxylgruppenhaltigen Verbindungen > 1:1 bis 1,3:1, bevorzugt 1,05:1 bis 1,2:1. Die Folgereaktion des isocyanatgruppenhaltigen Polyurethanpräpolymers zu dem erfindungsgemäß eingesetzten Urethan(meth)acrylat erfolgt vorzugsweise ebenfalls in einem der zuvor genannten Lösungsmittel. Gewünschtenfalls kann die Umsetzung jedoch ohne Lösungsmittel erfolgen. Die Temperatur liegt im Allgemeinen in einem Bereich von etwa 0 bis 60 °C, bevorzugt etwa 10 bis 50 °C**.** Die amingruppenhaltigen Komponenten a), c), d), e) und/oder f) werden im Allgemeinen in einer Menge eingesetzt, dass die freien Isocyanatgruppen des Polyurethanpräpolymers zumindest teilweise, vorzugsweise jedoch vollständig umgesetzt werden. Gegebenenfalls noch vorhandene Isocyanatgruppen werden abschließend durch Zusatz von Alkoholen, z. B. Ethanol, Aminen, z. B. 2-Amino-2-methyl-1-propanol, oder Mischungen davon inaktiviert.

Die Herstellung von Urethan(meth)acrylaten, die keine hydroxylgruppenhaltigen Komponenten eingebaut enthalten, erfolgt durch Umsetzung der amingruppenhaltigen Komponenten mit den isocyanatgruppenhaltigen Komponenten bei einer Temperatur im Bereich von etwa 0 bis 60 °C. Dabei können sowohl die Isocyanatkomponenten, als auch die amingruppenhaltigen Komponenten zur Umsetzung vorgelegt werden. Geeignete Lösungsmittel für diese Umsetzung sind z. B. Wasser, C₁-C₄-Alkohole, wie Methanol, n-Propanol, Isopropanol, n-Butanol und bevorzugt Ethanol und die zuvor genannten Ketone.

Vorzugsweise beträgt nach dem erfindungsgemässen Verfahren der Anteil an hydroxylgruppenhaltigen Komponenten a), c), d), e) und/oder f), bezogen auf die Gesamtmenge dieser Komponenten 0 bis 10 Gew.-%, bevorzugt 0 bis 5 Gew.-%, insbesondere 0,01 bis 1 Gew.-%.

Vorteilhafterweise eignen sich die in dem erfindungsgemässen Verfahren eingesetzten Lösungsmittel im Allgemeinen auch für die im Folgenden beschriebene Copolymerisation der Urethan(meth)acrylate zur Herstellung von wasserlöslichen oder wasserdispergierbaren Polymeren. Auf einen Lösungsmittelaustausch kann somit im Allgemeinen verzichtet werden. Vorteilhafterweise eignen sich die in dem erfindungsgemässen Verfahren eingesetzten Lösungsmittel im Allgemeinen auch zur Herstellung von Formulierungen dieser wasserlöslichen oder wasserdispergierbaren Polymere.

Säuregruppen enthaltende Urethan(meth)acrylate können durch teilweise oder vollständige Neutralisation mit einer Base, Aminogruppen enthaltende durch Neutralisation mit einer Säure oder durch Quaternisierung, in eine wasserlösliche bzw. wasserdispergierbare Form überführt werden. In der Regel weisen die erhaltenen Salze der Urethan(meth)acrylate eine bessere Wasserlöslichkeit oder Dispergierbarkeit in Wasser auf als die nicht neutralisierte Form. Geeignete Basen für die Neutralisation säuregruppenhaltiger Urethan(meth)acrylate und Säuren bzw Quaternisierungsmittel für die Protonierung oder Quaternisierung amingruppenhaltiger Urethan (meth) acrylate werden im Folgenden bei der Neutralisation bzw. Quaternisierung der Polymere auf Basis der erfindungsgemäßen Urethan (meth) acrylate genannt.

Gewünschtenfalls können die erfindungsgemäß eingesetzten Urethan (meth) acrylate auch in nicht neutralisierter bzw. nicht quaternisierter Form zur Umsetzung mit radikalisch polymerisierbaren Verbindungen, wie im Folgenden beschrieben, eingesetzt werden.

Ein weiterer Gegenstand der Erfindung sind radikalisch polymerisierbare, siloxangruppenhaltige Urethan(meth)acrylate, wie zuvor definiert, die als Komponente a) tert.-Butyl-aminoethylacrylat und/oder tert.-Butylaminoethylmethacrylat eingebaut enthalten, und die Salze davon.

Die zuvor beschriebenen, radikalisch polymerisierbaren, siloxangruppenhaltigen urethan(meth)acrylate können als Komponente zur Herstellung von Polymeren verwendet werden. Somit ist es möglich, siloxangruppenhaltige Polymere durch radikalische Copolymerisation der Urethan(meth)acrylate mit wenigstens einer weiteren Verbindung herzustellen, welche ebenfalls mindestens eine radikalisch polymerisierbare, α,β-ethylenisch ungesättigte Doppelbindung aufweist. Bei dieser weiteren Komponente kann es sich um wenigstens ein radikalisch polymerisierbares Monomer, Oligomer und/oder Polymer und Gemische davon handeln.

Ein weiterer Gegenstand der Erfindung ist ein wasserlösliches oder wasserdispergierbares Polymer, das wenigstens ein erfindungsgemäß eingesetztes Urethan (meth) acrylat, das als Komponente a) tert.-Butylaminoethylacrylat und/oder tert.-Butylaminoethylmethacrylat eingebaut enthält, und wenigstens ein radikalisch polymerisierbares, α,β-ethylenisch ungesättigtes Monomer M) einpolymerisiert enthält.

Die erfindungsgemäßen bzw. erfindungsgemäß eingesetzten wasserlöslichen oder wasserdispergierbaren Polymere enthalten die Urethan(meth)acrylate im Allgemeinen in einer Menge von etwa 0,05 bis 80 Gew.-%, bevorzugt 0,1 bis 50 Gew.-%, insbesondere 0,5 bis 35 Gew.-%, bezogen auf die Gesamtmenge von Urethan(meth)acrylat und Monomer M), einpolymerisiert.

Vorzugsweise ist das Monomer M) ausgewählt unter
M1) im Wesentlichen hydrophoben, nichtionischen Verbindungen, bevorzugt Estern α,β-ethylenisch ungesättigter Mono- und/oder Dicarbonsäuren mit C₁-C₃₀-Alkanolen, Amiden α,β-ethylenisch ungesättigter Mono- und/oder Dicarbonsäuren mit Mono- und Di-C₁-C₃₀-alkylaminen, Estern von Vinylalkohol oder Allylalkohol mit C₁-C₃₀-Monocarbonsäuren, Vinylethern, Vinylaromaten; Vinylhalogeniden, Vinylidenhalogeniden, C₂-C₈-Monoolefinen, nichtaromatischen Kohlenwasserstoffen mit mindestens 2 konjugierten Doppelbindungen und Mischungen davon,
M2) Verbindungen mit einer radikalisch polymerisierbaren, α,β-ethylenisch ungesättigten Doppelbindung und mindestens einer ionogenen und/oder ionischen Gruppe pro Molekül,
M3) im Wesentlichen hydrophilen, nichtionischen Verbindungen, bevorzugt N-Vinylamiden, N-Vinyllactamen, primären Amiden α,β-ethylenisch ungesättigter Monocarbonsäuren, vinyl- und allylsubstituierten heteroaromatischen Verbindungen, Estern α,β-ethylenisch ungesättigter Mono- und Dicarbonsäuren mit C₁-C₃₀-Alkandiolen, Estern und Amiden α,β-ethylenisch ungesättigter Mono- und Dicarbonsäuren mit C₂-C₃₀-Aminoalkoholen, die eine primäre oder sekundäre Aminogruppe aufweisen, Polyetheracrylaten, und Mischungen davon,
und Mischungen davon.

Geeignete Monomere M1) sind im Wesentlichen hydrophobe, nichtionische Monomere. Dazu zählen z. B. Vinylformiat, Vinylacetat, Vinylpropionat, Vinyl-n-butyrat, Vinylstearat, Vinyllaurat, Styrol, α-Methylstyrol, o-Chlorstyrol, Vinyltoluole, Vinylchlorid, Vinylidenchlorid, Ethylen, Propylen, Butadien, Isopren, Chloropren, Methyl-, Ethyl-, Butyl-, Dodecylvinylether etc.

Bevorzugte Monomere M1) sind z. B. die Ester α,β-ethylenisch ungesättigter Mono- und Dicarbonsäuren mit C₁-C₃₀-Alkanolen, bevorzugt C₁-C₂₂-Alkanolen. Bevorzugt sind weiterhin die Amide a,β-ethylenisch ungesättigter Mono- und Dicarbonsäuren mit Mono- und Dialkylaminen, die 1 bis 30 Kohlenstoffatome, bevorzugt 1 bis 22 Kohlenstoffatome, pro Alkylrest aufweisen. Vorzugsweise handelt es sich um Verbindungen der allgemeinen Formel V 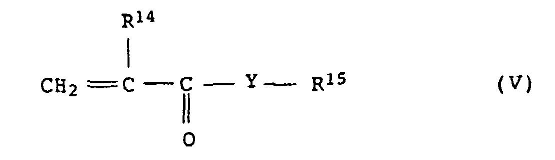 worin
- R¹⁴: für Wasserstoff oder C₁- bis C₈-Alkyl steht,
- R¹⁵: für einen geradkettigen oder verzweigten C₁- bis C₃₀-Alkylrest steht, und
- Y: für O oder NR¹⁶ steht, wobei R¹⁶ für Wasserstoff, C₁- bis C₈-Alkyl oder C₅- bis C₈-Cycloalkyl steht.

Vorzugsweise steht in der Formel V R¹⁴ für Wasserstoff, Methyl oder Ethyl.

Bevorzugt steht Y für O oder NH.

Geeignete Reste R¹⁵ sind die zuvor genannten C₁-C₃₀-Alkylreste. Insbesondere steht R¹⁵ für Methyl, Ethyl, n-Propyl, Isopropyl, n-Butyl, 2-Butyl, sec.-Butyl, tert.-Butyl, n-Pentyl, n-Hexyl, n-Heptyl, n-Octyl, Ethylhexyl, 1,1,3,3-Tetramethylbutyl, Undecyl, Lauryl, Tridecyl, Myristyl, Pentadecyl, Palmityl, Margarinyl, Stearyl, Palmitoleinyl, Oleyl oder Linolyl.

Insbesondere ist das Monomer M1) ausgewählt unter Methyl(meth)acrylat, Methylethacrylat, Ethyl(meth)acrylat, Ethylethacrylat, tert.-Butyl(meth)acrylat, tert.-Butylethacrylat, n-Octyl(meth)acrylat, 1,1,3,3-Tetramethylbutyl(meth)acrylat, Ethylhexyl(meth)acrylat, n-Nonyl(meth)acrylat, n-Decyl(meth)acrylat, n-Undecyl(meth)acrylat, Tridecyl(meth)acrylat, Myristyl(meth)acrylat, Pentadecyl(meth)acrylat, Palmityl(meth)acrylat, Heptadecyl(meth)acrylat, Nonadecyl(meth)acrylat, Arrachinyl(meth)acrylat, Behenyl(meth)acrylat, Lignocerenyl(meth)acrylat, Cerotinyl(meth)acrylat, Melissinyl(meth)acrylat, Palmitoleinyl(meth)acrylat, Oleyl(meth)acrylat, Linolyl(meth)acrylat, Linolenyl(meth)acrylat, Stearyl(meth)acrylat, Lauryl(meth)acrylat, tert.-Butyl(meth)acrylamid, n-Octyl(meth)acrylamid, 1,1,3,3-Tetramethylbutyl(meth)acrylamid, Ethylhexyl(meth)acrylamid, n-Nonyl(meth)acrylamid, n-Decyl(meth)acrylamid, n-Undecyl(meth)acrylamid, Tridecyl(meth)acrylamid, Myristyl(meth)acrylamid, Pentadecyl(meth)acrylamid, Palmityl(meth)acrylamid, Heptadecyl(meth)acrylamid, Nonadecyl(meth)acrylamid, Arrachinyl(meth)acrylamid, Behenyl(meth)acrylamid, Lignocerenyl(meth)acrylamid, Cerotinyl(meth)acrylamid, Melissinyl(meth)acrylamid, Palmitoleinyl(meth)acrylamid, Oleyl(meth)acrylamid, Linolyl(meth)acrylamid, Linolenyl(meth)acrylamid, Stearyl(meth)acrylamid, Lauryl(meth)acrylamid und Mischungen davon.

Bevorzugte Monomere M1) sind die Ester α,β-ethylenisch ungesättigter Mono- und Dicarbonsäuren mit geradkettigen und/oder verzweigter C₁-C₆-Alkanolen, bevorzugt C₂-C₄-Alkanolen, z. B. die Ester der Acrylsäure und/oder Methacrylsäure mit Methanol, Ethanol, n-Propanol, Isopropanol, n-Butanol, sec.-Butanol, tert.-Butanol, n-Pentanol, 2-Methylbutanol, n-Hexanol etc.

Bevorzugte Monomere M1) sind weiterhin Amide α,β-ethylenisch ungesättigter Mono- und Dicarbonsäuren mit Mono- und Dialkylaminen mit geradkettigen und/oder verzweigten Alkylresten, die 1 bis 6 Kohlenstoffatome, bevorzugt 2 bis 4 Kohlenstoffatome, pro Alkylrest aufweisen. Dazu zählen z. B. N-C₁- bis C₆-Alkyl(meth)acrylamide, wie N-Methyl(meth) acrylamid, N-Ethyl(meth)- acrylamid, N-(n-Propyl)(meth)acrylamid, N-Isopropyl(meth)acrylamid, N-(n-Butyl)(meth)acrylamid, N-(tert.-Butyl)(meth)acrylamid, N-(n-Pentyl)(meth)acrylamid, N-(n-Hexyl)(meth)acrylamid, N,N-Dimethyl(meth)acrylamid, N,N-Diethyl(meth)acrylamid etc.

Vorzugsweise umfasst die Komponente M1) wenigstens einen Ester einer α,β-ethylenisch ungesättigten Mono- und/oder Dicarbonsäure mit einem linearen C₂- bis C₆-Alkanol. Insbesondere handelt es sich um einen Ester der Acrylsäure und/oder Methacrylsäure mit Ethanol, n-Propanol, n-Butanol, n-Pentanol und n-Hexanol. Speziell umfasst die Komponente M1) n-Butylacrylat und/oder n-Butylmethacrylat.

Vorzugsweise umfasst die Komponente M1) wenigstens einen Ester einer α,β-ethylenisch ungesättigten Mono- und/oder Dicarbonsäure mit einem verzweigten C₂- bis C₆-Alkanol. Speziell umfasst die Komponente M1) tert.-Butylacrylat und/oder tert.-Butylmethacrylat.

Vorzugsweise umfasst die Komponente M1) mindestens ein lineares C₁- bis C₆-Alkyl(meth)acrylat und/oder -acrylamid, insbesondere n-Butyl(meth)acrylat und/oder n-Butyl(meth)acrylamid,

Vorzugsweise umfasst die Komponente M1) mindestens ein verzweigtes C₁- bis C₆-Alkyl(meth)acrylat und/oder -acrylamid, insbesondere tert.-Butyl(meth)acrylat und/oder tert.-Butyl(meth)acrylamid.

Besonders bevorzugt wird als Komponente M1) ein Monomerengemisch eingesetzt, das mindestens eines der zuvor genannten linearen C₁- bis C₆-Alkyl(meth)acrylate und/oder -acrylamide und mindestens eines der zuvor genannten verzweigten C₁- bis C₆-Alkyl(meth)acrylate und/oder -acrylamide umfasst.

Die Verbindungen M2) weisen mindestens eine ionogene bzw. ionische Gruppe pro Molekül auf, die bevorzugt ausgewählt ist unter Carboxylatgruppen und/oder Sulfonatgruppen und deren durch teilweise oder vollständige Neutralisation mit einer Base erhältlichen Salze, sowie tertiäre Amingruppen, die teilweise oder vollständig protoniert und quaternisiert sein können. Geeignete Basen für die Neutralisation bzw. Säuren für die Protonierung und Alkylierungsmittel für die Quaternisierung sind die zuvor genannten.

Geeignete Monomere M2) sind z. B. die zuvor genannten, α,β-ethylenisch ungesättigten Mono- und/oder Dicarbonsäuren und deren Halbester und Anhydride, wie Acrylsäure, Methacrylsäure, Fumarsäure, Maleinsäure, Itaconsäure, Crotonsäure, Maleinsäureanhydrid, Monobutylmaleat etc. Bevorzugt werden Acrylsäure, Methacrylsäure und deren Alkalimetallsalze, wie deren Natrium- und Kaliumsalze, eingesetzt.

Geeignete Monomere M2) sind weiterhin Acrylamidoalkansulfonsäuren und deren Salze, wie 2-Acrylamido-2-methylpropansulfonsäure und deren Alkalimetallsalze, z. B. deren Natrium- und Kaliumsalze.

Weitere geeignete Verbindungen M2) sind die Ester der zuvor genannten, α,β-ethylenisch ungesättigten Mono- und Dicarbonsäuren mit C₂- bis C₁₂-Aminoalkoholen, welche am Aminstickstoff C₁- bis C₈-dialkyliert sind. Dazu zählen z. B. N,N-Dimethylaminomethyl-(meth)acrylat, N,N-Dimethylaminoethyl(meth)acrylat, N,N-Diethylaminoethyl(meth)acrylat, N,N-Dimethylaminopropyl(meth)acrylat, N,N-Diethylaminopropyl(meth)acrylat, N,N-Dimethylaminocyclohexyl(meth)acrylat etc. Bevorzugt werden N,N-Dimethylaminopropylacrylat und N,N-Dimethylaminopropyl(meth)acrylat eingesetzt.

Geeignete Monomere M2) sind weiterhin die Amide der zuvor genannten α,β-ethylenisch ungesättigten Mono- und Dicarbonsäuren mit Diaminen, die eine tertiäre und ein primäre oder sekundäre Aminogruppe aufweisen. Dazu zählen z. B. N-[2-(dimethylamino)ethyl]-acrylamid, N-[2-(dimethylamino)ethyl]methacrylamid, N-[3-(dimethylamino) propyl]acrylamid, N-[3-(dimethylamino)propyl]methacrylamid, N-(4-(dimethylamino)butyl]acrylamid, N-[4-(dimethylamino)-butyl]methacrylamid, N-[2-(diethylamino)ethyl]acrylamid, N-[4-(dimethylamino)cyclohexyl]acrylamid, N-[4-(dimethylamino)cyclohexyl]methacrylamid etc.

Geeignete Monomere M3) sind im Wesentlichen hydrophile, nichtionische Monomere. Dazu zählen z. B. N-vinylamide, wie N-Vinylformamid, N-Vinylacetamid, N-Vinylpropionamid etc. Bevorzugt wird N-Vinylformamid eingesetzt.

Geeignete Monomere M3) sind weiterhin N-Vinyllactame und deren Derivate, die z. B. einen oder mehrere C₁-C₆-Alkylsubstituenten, wie Methyl, Ethyl, n-Propyl, Isopropyl, n-Butyl, sec.-Butyl, tert.-Butyl etc. aufweisen können. Dazu zählen z. B. N-Vinylpyrrolidon, N-Vinylpiperidon, N-Vinylcaprolactam, N-Vinyl-5-methyl-2-pyrrolidon, N-Vinyl-5-ethyl-2-pyrrolidon, N-Vinyl-6-methyl-2-piperidon, N-Vinyl-6-ethyl-2-piperidon, N-Vinyl-7-methyl-2-caprolactam, N-Vinyl-7-ethyl-2-caprolactam etc.

Geeignete Monomere M3) sind weiterhin primäre Amide der zuvor genannten α,β-ethylenisch ungesättigten Monocarbonsäuren, wie Acrylamid, Methacrylamid, Ethacrylamid etc.

Geeignete Monomere M3) sind weiterhin vinyl- und allylsubstituierte heteroaromatische Verbindungen, wie 2- und 4-Vinylpyridin, -Allylpyridin, und bevorzugt N-Vinylheteroaromaten, wie N-Vinylimidazol, N-Vinyl-2-methylimidazol etc.

Geeignete Monomere M3) sind weiterhin die Ester α,β-ethylenisch ungesättigter Mono- und Dicarbonsäuren, wie Acrylsäure, Methacrylsäure, Fumarsäure, Maleinsäure, Itaconsäure, Crotonsäure etc., mit C₁-C₃₀-Alkandiolen. Dazu zählen z. B. 2-Hydroxyethylacrylat, 2-Hydroxyethylmethacrylat, 2-Hydroxyethylethacrylat, 2-Hydroxypropylacrylat, 2-Hydroxypropylmethacrylat, 3-Hydroxypropylacrylat, 3-Hydroxypropylmethacrylat, 3-Hydroxybutylacrylat, 3-Hydroxybutylmethacrylat, 4-Hydroxybutylacrylat, 4-Hydroxybutylmethacrylat, 6-Hydroxyhexylacrylat, 6-Hydroxyhexylmethacrylat, 3-Hydroxy-2-ethylhexylacrylat, 3-Hydroxy-2-ethylhexylmethacrylat etc. Vorzugsweise werden Hydroxyethylacrylat und Hydroxyethylmethacrylat eingesetzt. Geeignete Monomere e) sind auch die Ester der zuvor genannten Säuren mit Triolen und Polyolen, wie z. B. Glycerin, Erythrit, Pentaerythrit, Sorbit etc.

Geeignete Verbindungen M3) sind weiterhin Polyetheracrylate der allgemeinen Formel VI 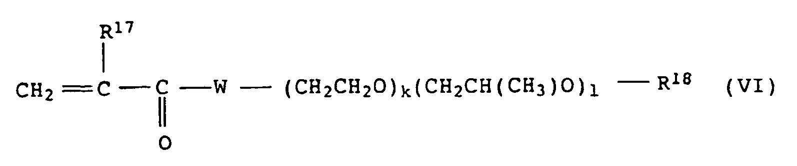 worin
die Reihenfolge der Alkylenoxideinheiten beliebig ist,
- k und l: unabhängig voneinander für eine ganze Zahl von 0 bis 50 stehen, wobei die Summe aus k und 1 mindestens 5 beträgt,
- R¹⁷: für Wasserstoff oder C₁- bis C₈-Alkyl steht, und
- R¹⁸: für Wasserstoff oder C₁- bis C₆-Alkyl steht,
- W: für O oder NR¹⁹ steht, wobei R¹⁹ für Wasserstoff, C₁- bis C₈-Alkyl oder C₅- bis C₈-Cycloalkyl steht.

Bevorzugt handelt es sich bei den Polyetheracrylaten M3) um Verbindungen der allgemeinen Formel VI, worin die Summe aus k und 1 für eine ganze Zahl von 5 bis 70, bevorzugt 6 bis 50, steht.

In der Formel VI steht R¹⁷ bevorzugt für Wasserstoff Methyl, Ethyl, n-Propyl, Isopropyl, n-Butyl, sec.-Butyl, tert.-Butyl, n-Pentyl oder n-Hexyl, insbesondere für Wasserstoff, Methyl oder Ethyl.

Vorzugsweise steht R¹⁸ in der Formel VI für Wasserstoff, Methyl, Ethyl, n-Propyl, Isopropyl, n-Butyl, sec-Butyl, n-Pentyl oder n-Hexyl.

Vorzugsweise steht W in der Formel VI für O oder NH.

Geeignete Polyetheracrylate M3) sind z. B. die Polykondensationsprodukte der zuvor genannten α,β-ethylenisch ungesättigten Mono- und/oder Dicarbonsäuren und deren Säurechloriden, -amiden und Anhydriden mit Polyetherolen. Geeignete Polyetherole können leicht durch Umsetzung von Ethylenoxid, 1,2-Propylenoxid und/oder Epichlorhydrin mit einem Startermolekül, wie Wasser oder einem kurzkettigen Alkohol R¹⁶-OH hergestellt werden. Die Alkylenoxide können einzeln, alternierend nacheinander oder als Mischung eingesetzt werden. Die Polyetheracrylate M3) können allein oder in Mischungen zur Herstellung der erfindungsgemäß eingesetzten Polymere verwendet werden.

Nach einer ersten bevorzugten Ausführungsform enthalten die erfindungsgemäßen bzw. erfindungsgemäß eingesetzten Wasserlöslichen oder wasserdispergierbaren Polymere wenigstens ein erfindungsgemäß eingesetztes Urethan(meth)acrylat, wie zuvor beschrieben, wenigstens eine im Wesentlichen hydrophobe, nichtionische Verbindung M1) und wenigstens eine ionogene bzw. ionische Verbindung M2) eingebaut. Gegebenenfalls können diese Polymere zusätzlich wenigstens eine weitere Verbindung der Komponente M3) einpolymerisiert enthalten.

Vorzugsweise enthält das Polymer
- 0,1 bis 50 Gew.-%, bevorzugt 0,5 bis 35 Gew.-%, wenigstens eines erfindungsgemässen Urethan(meth)acrylats,
- 40 bis 75 Gew.-%, bevorzugt 45 bis 73 Gew.-%, wenigstens einer Komponente M1),
- 10 bis 35 Gew.-%, bevorzugt 18 bis 30 Gew.-%, wenigstens einer Komponente M2),
- 0 bis 30 Gew.-%, wenigstens einer Komponente M3),
einpolymerisiert.

Besonders bevorzugt sind Polymere, die
- 0,1 bis 50 Gew.-%, bevorzugt 0,5 bis 35 Gew.-%, wenigstens eines erfindungsgemässen Urethan(meth)acrylats ,
- 40 bis 75 Gew.-%, bevorzugt 45 bis 73 Gew.-%, wenigstens einer Komponente M1), die ausgewählt ist unter linearen und verzweigten C₁- bis C₆-Alkyl(meth)acrylaten, C₁- bis C₆-Alkyl(meth)acrylamiden und Gemischen davon,
- 10 bis 35 Gew.-%, bevorzugt 18 bis 30 Gew.-%, wenigstens einer α,β-ethylenisch ungesättigten Mono- und/oder Dicarbonsäure M2), bevorzugt Acrylsäure, Methacrylsäure und Gemische davon,
- 0 bis 30 Gew.-%, bevorzugt 0,1 bis 25 Gew.-% wenigstens einer Komponente M3),
einpolymerisiert enhalten.

Besonders bevorzugt sind weiterhin Polymere, die
- 0,1 bis 50 Gew.-%, bevorzugt 0,5 bis 35 Gew.-%, wenigstens eines erfindungsgemäß eingesetzten Urethan(meth)acrylats,
- 40 bis 75 Gew.-%, bevorzugt 45 bis 73 Gew.-%, wenigstens einer Komponente M1), die ausgewählt ist unter linearen und verzweigten C₁- bis C₆-Alkyl(meth)acrylaten, C₁- bis C₆-Alkyl(meth)acrylamiden und Gemischen davon,
- 10 bis 35 Gew.-%, bevorzugt 18 bis 30 Gew.-%, wenigstens einer Komponente M2), die ausgewählt ist unter Estern der zuvor genannten, α,β-ethylenisch ungesättigten Mono- und Dicarbonsäuren mit C₂- bis C₁₂-Aminoalkoholen, welche am Aminstickstoff C₁- bis C₈-dialkyliert sind, Amiden der zuvor genannten α,β-ethylenisch ungesättigten Mono- und Dicarbonsäuren mit Diaminen, die eine tertiäre und ein primäre oder sekundäre Aminogruppe aufweisen, und Mischungen davon,
- 0 bis 30 Gew.-%, bevorzugt 0,1 bis 25 Gew.-% wenigstens einer Komponente M3),
einpolymerisiert enhalten.

Nach einer weiteren bevorzugten bevorzugten Ausführungsform enthalten die erfindungsgemäßen bzw. erfindungsgemäß eingesetzten wasserlöslichen oder wasserdispergierbaren Polymere wenigstens ein erfindungsgemäß eingesetztes Urethan(meth)acrylat, wie zuvor beschrieben,und wenigstens eine im Wesentlichen hydrophile, nichtionische Verbindung M3) eingebaut. Gegebenenfalls können diese Polymere zusätzlich wenigstens eine weitere Verbindung, die ausgewählt ist unter den Verbindungen der Komponenten M1) und/oder M2), einpolymerisiert enthalten.

Vorzugsweise enthält das Polymer
- 0,1 bis 50 Gew.-%, bevorzugt 0,5 bis 35 Gew.-%, wenigstens eines erfindungsgemässen Urethan(meth)acrylats,
- 0 bis 50 Gew.-% wenigstens einer Komponente M1),
- 0 bis 20 Gew.-%, wenigstens einer Komponente M2),
- 25 bis 80 Gew.-%, wenigstens einer Komponente M3), einpolymerisiert.
   Besonders bevorzugt sind Polymere, die
- 0,1 bis 50 Gew.-%, bevorzugt 0,5 bis 35 Gew.-%, wenigstens eines erfindungsgemäß eingesetzten Urethan(meth)acrylats,
- 0 bis 50 Gew.-%, bevorzugt 0,1 bis 45 Gew.-%, wenigstens einer Komponente M1), die ausgewählt ist unter linearen und verzweigten C₁- bis C₆-Alkyl(meth)acrylaten, C₁- bis C₆-Alkyl(meth)acrylamiden und Gemischen davon,
- 0 bis 20 Gew.-%, bevorzugt 0,1 bis 18 Gew.-%, wenigstens einer Komponente M2), die ausgewählt ist unter Estern der zuvor genannten, α,β-ethylenisch ungesättigten Mono- und Dicarbonsäuren mit C₂- bis C₁₂-Aminoalkoholen, welche am Aminstickstoff C₁- bis C₈-dialkyliert sind, Amiden der zuvor genannten α,β-ethylenisch ungesättigten Mono- und Dicarbonsäuren mit Diaminen, die eine tertiäre und ein primäre oder sekundäre Aminogruppe aufweisen, und Mischungen davon,
- 25 bis 80 Gew.-%, wenigstens einer Komponente M3), die ausgewählt ist unter N-Vinyllactamen und deren Derivaten, bevorzugt N-Vinylpyrrolidon und/oder N-Vinylcaprolactam,
einpolymerisiert enthalten.

Vorzugsweise enthalten die Polymere als Komponente M1) mindestens ein lineares C₁- bis C₆-Alkyl(meth)acrylat und/oder -acrylamid, insbesondere n-Butyl(meth)acrylat und/oder n-Butyl(meth)acrylamid, und mindestens ein verzweigtes C₁- bis C₆-Alkyl(meth)acrylat und/oder -acrylamid, insbesondere tert.-Butyl(meth)acrylat und/oder tert.-Butyl(meth)acrylamid, einpolymerisiert.

Vorzugsweise weisen die erfindungsgemäßen bzw. erfindungemäß eingesetzten Polymere als Komponente M2) wenigstens eine Verbindung mit mindestens einer anionogenen und/oder anionischen Gruppe pro Molekül auf. Die Säurezahl dieser Polymere liegt dann bevorzugt in einem Bereich von 30 bis 190 mg KOH/g.

Vorzugsweise weisen die erfindungsgemäßen bzw. erfindungsgemäß eingesetzten Polymere als Komponente M2) wenigstens eine Verbindung mit mindestens einer kationogenen und/oder kationischen Gruppe pro Molekül auf. Bevorzugt liegt die Aminzahl bzw. die quaternäre Ammoniumzahl der erfindungsgemäßen Polymere dann in einem Bereich von etwa 30 bis 190 mg KOH/g.

Die Herstellung der erfindungsgemäßen bzw. erfindungsgemäß eingesetzten Polymere erfolgt durch radikalische Polymerisation nach üblichen, dem Fachmann bekannten Verfahren. Dazu zählt die radikalische Polymerisation in Masse, Emulsion, Suspension und in Lösung, vorzugsweise die Emulsions- und Lösungspolymerisation. Die Mengen an zu polymerisierenden Verbindungen, bezogen auf Lösungs- bzw. Dispergiermittel, werden dabei im Allgemeinen so gewählt, dass etwa 30 bis 80 Gew.-% Lösungen, Emulsionen oder Dispersionen erhalten werden. Die Polymerisationstemperatur beträgt in der Regel 30 bis 120 °C, bevorzugt 40 bis 100 °C. Das Polymerisationsmedium für die Lösungspolymerisation kann sowohl nur aus einem organischen Lösungsmittel als auch aus Mischungen aus Wasser und mindestens einem wassermischbaren, organischen Lösungsmittel bestehen. Bevorzugte organische Lösungsmittel sind z. B. Alkohole, wie Methanol, Ethanol, n-Propanol, Isopropanol, n-Butanol, Ketone, wie Aceton und Methylethylketonen, Tetrahydrofuran etc. Die Lösungspolymerisation kann sowohl als Batchprozess_als auch in Form eines Zulaufverfahrens, einschließlich Monomerenzulauf, Stufen- und Gradientenfahrweise, durchgeführt werden. Bevorzugt ist im Allgemeinen das Zulaufverfahren, bei dem man gegebenenfalls einen Teil des Polymerisationsansatzes vorlegt, auf die Polymerisationstemperatur erhitzt und anschließend den Rest des Polymerisationsansatzes, üblicherweise über einen oder auch mehrere, räumliche getrennte Zuläufe, kontinuierlich, stufenweise oder unter Überlagerung eines Konzenträtionsgefälles unter Aufrechterhaltung der Polymerisation der Polymerisationszone zuführt.

Als Initiatoren für die radikalische Polymerisation werden übliche Peroxo- oder Azoverbindungen eingesetzt. Dazu zählen z.B. Dibenzoylperoxid, tert.-Butylperpivalat, tert.-Butylper-2-ethylhexanoat, Di-tert.-butylperoxid, 2,5-Dimethyl-2,5-di(tert.-butylperoxy)hexan, aliphatische oder cycloaliphatische Azoverbindungen, z. B. 2,2'-Azobis(isobutyronitril), 2,2'-Azobis(2-methylbutyronitril), 2,2'-Azobis(2,4-dimethylvaleronitril), 1,1'-Azobis(1-cyclohexancarbonitril), 2-(Carbamoylazo)isobutyronitril, 4,4' -Azobis (4-cyanovaleriansäure) und deren Alkalimetall- und Ammoniumsalze, z. B. das Natriumsalz, Dimethyl-2,2'-azobisisobutyrat, 2,2'-Azobis[2-(2-imidazolin-2-yl)propan], 2,2'-Azobis(2-amidinopropan) und die Säureadditionssalze der beiden zuletzt genannten Verbindungen, z. B. die Dihydrochloride.

Ferner kommen als Initiatoren Wasserstoffperoxid, Hydroperoxide in Kombination mit Reduktionsmitteln und Persalze in Frage. Geeignete Hydroperoxide sind beispielsweise t-Butylhydroperoxid, t-Amylhydroperoxid, Cumolhydroperoxid und Pinanhydroperoxid jeweils in Kombination mit beispielsweise einem Salz der Hydroxymethansulfinsäure, einem Eisen (II)-Salz oder Ascorbinsäure. Geeignete Persalze sind insbesondere Alkalimetallperoxidisulfate.

Die verwendete Initiatormenge, bezogen auf die Monomere, liegt im Allgemeinen in einem Bereich von etwa 0,1 bis 2 Gew.-% bezogen auf das Gesamtgewicht der zu polymerisierenden Monomere.

Die K-Werte der resultierenden Copolymerisate liegen vorzugsweise in einem Bereich von etwa 15 bis 90, bevorzugt 20 bis 70, insbesondere 25 bis 60 (1 gew.-%ige Lösung in Ethanol). Zur Erzielung des gewünschten K-Wertes kann, insbesondere bei der Emulsions- oder Suspensionspolymerisation, der Einsatz eines Reglers angebracht sein. Als Regler eignen sich beispielsweise Aldehyde, wie Formaldehyd, Acetaldehyd, Propionaldehyd, n-Butyraldehyd und Isobutyraldehyd, Ameisensäure, Ammoniumformiat, Hydroxylammoniumsulfat und Hydroxylammoniumphosphat. Weiterhin können Regler eingesetzt werden, die Schwefel in organisch gebundener Form enthalten, wie Di-n-butylsulfid, Di-n-octylsulfid, Diphenylsulfid etc., oder Regler, die Schwefel in Form von SH-Gruppen enthalten, wie n-Butylmercaptan, n-Hexylmercaptan oder n-Dodecylmercaptan. Geeignet sind auch wasserlösliche, schwefelhaltige Polymerisationsregler, wie beispielsweise Hydrogensulfite und Disulfite. Weiterhin eignen sich als Regler Allylverbindungen, wie Allylalkohol oder Allylbromid, Benzylverbindungen, wie Benzylchlorid oder Alkylhalogenide, wie Chloroform oder Tetrachlormethan.

Gewünschtenfalls setzt man der Polymerlösung im Anschluss an die Polymerisationsreaktion einen oder mehrere Polymerisationsinitiatoren zu und erhitzt die Polymerlösung, z. B. auf die Polymerisationstemperatur oder auf Temperaturen oberhalb der Polymerisationstemperatur, um die Polymerisation zu vervollständigen. Geeignet sind die oben angegebenen Azoinitiatoren, aber auch alle anderen üblichen, für eine radikalische Polymerisation in wässriger Lösung geeignete Initiatoren, beispielsweise Peroxide, Hydroperoxide, Peroxodisulfate, Percarbonate, Peroxoester und Wasserstoffperoxid. Hierdurch wird die Polymerisationsreaktion zu einem höheren Umsatz, wie z. B. von 99,9 %, geführt. Die bei der Polymerisation entstehenden Lösungen können gegebenenfalls durch ein dem Stand der Technik entsprechendes Trocknungsverfahren in feste Pulver überführt werden. Bevorzugte Verfahren sind beispielsweise die Sprühtrocknung, die Sprühwirbelschichttrocknung, die Walzentrocknung und die Bandtrocknung. Ebenfalls anwendbar sind die Ge-friertrocknung und die Gefrierkonzentrierung. Gewünschtenfalls kann das Lösungsmittel auch durch übliche Methoden, z. B. Destillation bei verringertem Druck, teilweise oder vollständig entfernt werden.

Nach einer geeigneten Ausführungsform handelt es sich bei den erfindungsgemäßen bzw. erfindungsgemäß eingesetzten Polymeren um nichtionische Polymere.

Nach einer weiteren geeigneten Ausführungsform handelt es sich bei den erfindungsgemäßen bzw. erfindungsgemäß eingesetzten wasserlöslichen oder wasserdispergierbaren Polymeren um anionische bzw. anionogene Polymere. Die Säuregruppen der Polymere können mit einer Base teilweise oder vollständig neutralisiert werden. In aller Regel weisen die erhaltenen Salze der Polymere eine bessere Wasserlöslichkeit oder Dispergierbarkeit in Wasser auf als die nicht neutralisierten Polymere. Als Base für die Neutralisation der Polymere können Alkalimetallbasen wie Natronlauge, Kalilauge, Soda, Natriumhydrogencarbonat, Kaliumcarbonat oder Kaliumhydrogencarbonat und Erdalkalimetallbasen wie Calciumhydroxyd, Calciumoxid, Magnesiumhydroxyd oder Magnesiumcarbonat sowie Ammoniak und Amine verwendet werden. Geeignete Amine sind z. B. C₁-C₆-Alkylamine, bevorzugt n-Propylamin und n-Butylamin, Dialkylamine, bevorzugt Diethylpropylamin und Dipropylmethylamin, Trialkylamine, bevorzugt Triethylamin und Triisopropylamin, C₁-C₆-Alkyldiethyanolamine, bevorzugt Methyl- oder Ethyldiethanolamin und Di-C₁-C₆-Alkylethanolamine. Besonders für den Einsatz in Haarbehandlungsmitteln haben sich zur Neutralisation der Säuregruppen enthaltenden Polymere 2-Amino-2-methyl-1-propanol, 2-Amino-2-ethylpropan-1,3-diol, Diethylaminopropylamin und Triisopropanolamin bewährt. Die Neutralisation der Säuregruppen enthaltenden Polymere kann auch mit Hilfe von Mischungen mehrerer Basen vorgenommen werden, z. B. Mischungen aus Natronlauge und Triisopropanolamin. Die Neutralisation kann je nach Anwendungszweck partiell z. B. zu 5 bis 95 %, vorzugsweise 30 bis 95 %, oder vollständig, d. h. zu 100 % erfolgen.

Nach einer weiteren geeigneten Ausführungsform handelt es sich bei den erfindungsgemäßen bzw. erfindungsgemäß eingesetzten wasserlöslichen oder wasserdispergierbaren Polymeren um kationische bzw. kationogene Polymere. Die Amingruppen bzw. protonierte oder quaternisierte Amingruppen enthaltenden Polymere sind aufgrund ihrer kationischen Gruppen im Allgemeinen leicht in Wasser oder Wasser/Alkohol-Gemischen löslich oder zumindest ohne Zuhilfenahme von Emulgatoren dispergierbar. Geladene kationische Gruppen lassen sich aus den vorliegenden tertiären Aminstickstoffen entweder durch Protonierung, z. B. mit Carbonsäuren, wie Milchsäure, oder Mineralsäuren, wie Phosphorsäure, Schwefelsäure und Salzsäure, oder durch Quaternisierung, z. B. mit Alkylierungsmitteln, wie C₁- bis C₄-Alkylhalogeniden oder -sulfaten, erzeugen. Beispiele solcher Alkylierungsmit- tel sind Ethylchlorid, Ethylbromid, Methylchlorid, Methylbromid, Dimethylsulfat und Diethylsulfat. ,

Nach einer weiteren geeigneten Ausführungsform können die erfindungsgemäßen bzw. erfindungsgemäß eingesetzten Polymere sowohl Säuregruppen als auch Aminogruppen aufweisen. Der Betrag der Differenz aus Säuregruppen und Aminogruppen (|ΔSZ-AZ|) liegt dabei vorzugsweise in einem Bereich von etwa 15 bis 150, bevorzugt 30 bis 100. Säurezahl und Aminzahl sind dabei jeweils als mg KOH/g Prüfsubstanz definiert.

Wird bei der Herstellung der Polyurethane ein wassermischbares organisches Lösungsmittel eingesetzt, so kann dieses im Anschluss durch übliche, dem Fachmann bekannte Verfahren, z. B. durch Destillation bei vermindertem Druck, entfernt werden. Vor dem Abtrennen des Lösungsmittels kann dem Polyurethan zusätzlich Wasser zugegeben werden. Nach Ersatz des Lösungsmittels durch Wasser erhält man eine Lösung oder Dispersion des Polymers, aus der, falls gewünscht, das Polymer in üblicher Weise gewonnen werden kann, z. B. durch Sprühtrocknung.

Die erfindungsgemäßen bzw. erfindungsgemäß eingesetzten Polymere weisen Siloxangruppen auf. Vorzugsweise beträgt der auf das Gesamtgewicht der eingebauten Komponenten bezogene Siloxangehalt etwa 0,05 bis 30 Gew.-%, bevorzugt 0,05 bis 25 Gew.-%, insbesondere 0,1 bis 20 Gew.-%. Ihre K-Werte (gemessen nach E. Fikentscher, Cellulose-Chemie 13 (1932), S. 58-64, an einer 1%igen Lösung in Ethanol) liegen im Allgemeinen in einem Bereich von etwa 15 bis 90, bevorzugt 20 bis 60. Ihre Glasübergangstemperatur beträgt im Allgemeinen mindestens 0 °C, bevorzugt mindestens 20 °C, insbesondere bevorzugte mindestens 25 °C und speziell mindestens 30 °C. Weisen die erfindungsgemäßen Polymere zwei oder mehrere Glasübergangstemperaturen auf, so liegt wenigstens eine davon in dem angegebenen Bereich. Bevorzugt liegt/liegen die andere(n) dann unterhalb des zuvor angegebenen Temperaturbereichs.

Ein weiterer Gegenstand der Erfindung ist die Verwendung von wasserlöslichen oder wasserdispergierbaren Polymeren, die ausgewählt sind unter erfindungsgemässen bzw. erfindungsgemäß eingesetzten Polymeren, die wenigstens ein siloxangruppenhaltiges Urethan(meth)acrylaten und wenigstens ein radikalisch polymerisierbares, α,β-ethylenisch ungesättigtes Monomer M) einpolymerisiert enthalten, wie zuvor beschrieben, und Mischungen davon, in der Haarkosmetik, bevorzugt als Festigerpolymer in Haarsprays, Schaumfestigern, Haarmousse, Haargel und Schampoos, in der Hautkosmetik, bevorzugt in Cremes, pigmenthaltigen Hautkosmetika und wachshaltigen Hautkosmetika.

Neben den erfindügsgemässen bzw. erfindungsgemäß eingesetzten Polymeren eignen sich vorzugsweise auch siloxangruppenfreie polymere und Mischungen von siloxangruppenhaltigen und siloxangruppenfreien Polymeren für die Verwendung in der Haarkosmetik, bevorzugt als Festigerpolymer in Haarsprays, Schaumfestigern, Haarmousse, Haargel und Schampoos.

Zur Herstellung von siloxangruppenfreien wasserlöslichen oder wasserdispergierbaren Polymeren wird wenigstens ein siloxangruppenfreies Urethan(meth)acrylat und wenigstens ein radikalisch polymerisierbares, α,β-ethylenisch ungesättigtes Monomer M) einer radikalischen copolymerisation unterzogen. Geeignete Polymerisationsverfahren sind die üblichen, dem Fachmann bekannten Verfahren. Dazu zählen die zuvor für die Herstellung der erfindungsgemässen bzw. erfindungsgemäß eingesetzten Polymere beschriebenen.

Die Herstellung geeigneter radikalisch polymerisierbarer, siloxangruppenfreier Urethan(meth)acrylate erfolgt, wie zuvor für die Herstellung der erfindungsgemässen bzw. erfindungsgemäß eingesetzten siloxangruppenhaltigen Urethan(meth)acrylate beschrieben. Dabei wird auf den Einsatz siloxangruppenhaltiger Komponenten d) verzichtet. Zur Berstellung geeignete bzw- bevorzugte Komponenten a), b), c), e), f) und g) sind die zuvor entsprechend genannten.

Vorzugsweise ist das Monomer M) ausgewählt unter den zuvor beschriebenen Monomeren M1), M2), M3) und Mischungen davon.

Bevorzugt verwendet man die siloxangruppenfreien Polymere in Haarbehandlungsmitteln, wie Schaumfestigern, Haarmousse, Haargel, Schampoos und insbesondere Haarsprays. Geeignete Komponenten und deren Einsatzmengen zur Formulierung von Haarbehandlungsmitteln sind die im Folgenden für Haarbehandlungsmittel auf Basis der erfindungsgemäßen bzw. erfindungsgemäß eingesetzten siloxangruppenhaltigen Polymere genannten.

Die erfindungsgemäßen bzw. erfindungsgemäß eingesetzten siloxangruppenhaltigen Polymere und die zuvor beschriebenen siloxangruppenfreien Polymere sind als Hilfsmittel in der Kosmetik, insbesondere als oder in Besehichtungsmittel(n) für keratinhaltige Oberflächen (Haar, Haut und Nägel) brauchbar. Sie sind insbesondere in der Haarkosmetik brauchbar. Die zuvor genannten Polymere können auch in Cremes verwendet werden. Sie eignen sich auch als Bindemittel und Klebemittel für kosmetische Produkte, z. B. bei der Herstellung stiftförmiger kosmetischer Produkte, wie Deostifte, Schminkstifte etc.

Die erfindungsgemäßen bzw. erfindungsgemäß eingesetzten siloxangruppenhaltigan Polymere und die zuvor beschriebenen siloxangruppenfreien Polymere eignen sich vorzugsweise für die Verwendung in der Hautkosmetik, bevorzugt in Cremes, pigmenthaltigen Hautkosmetika und wachsbaltigen Hautkosmetika.

Im Allgemeinen enthält das erfindungsgemäße Mittel die Polymere in einer Menge im Bereich von 0,2 bis 30 Gew.-%, bezogen auf das Gesamtgewicht des Mittels.

Die erfindungsgemäßen Mittel eignen sich insbesondere als Beschichtungsmittel für keratinhaltige Oberflächen (Haar, Haut und Nägel). Die in ihnen eingesetzten, gegebenenfalls neutralisierten bzw. quaternisierten Verbindungen sind wasserlöslich oder wasserdispergierbar. Sind die in den erfindungsgemäßen Mitteln eingesetzten Verbindungen wasserdisporgierbar, können sie in Form von wässrigen Mikrodispersionen mit Teilchendurchmessern von üblicherweise 1 bis 250 nm, bevorzugt 1 bis 150 nm, zur Anwendung gebracht werden. Die Feststoffgehalte der Präparate liegen dabei üblicherweise in einem Bereich von etwa 0,5 bis 20 Gew.-%, bevorzugt 1 bis 12 Gew.-%. Diese Mikrodispersionen benötigen in der Regel keine Emulgatoren oder Tenside zu ihrer Stabilisierung.

Die erfindungsgemäßen Mittel liegen in Form eines Haarbehandlungsmittels, wie Schaumfestiger, Haarmousse, Haargel, Schampoo und insbesondere in Form eines Haarsprays vor. Zur Anwendung als Haarfestiger sind dabei Mittel bevorzugt, die Polyurethane enthalten, die wenigstens eine Glasübergangstemperatur T_{g} ≥ 20°C, bevorzugt ≥ 30°C, aufweisen. Der K-Wert dieser Polymere liegt vorzugsweise in einem Bereich von 23 bis 90, insbesondere 25 bis 60. Der Siloxangehalt dieser Polymere beträgt im Allgemeinen 0,05 bis 20 Gew.-%, bezogen auf das Gesamtgewicht der eingebauten Komponenten.

Die erfindungsgemäßen Haarbehandlungsmittel liegen üblicherweise in Form einer wässrigen Dispersion oder in Form einer alkoholischen oder wässrig-alkoholischen Lösung vor. Beispiele geeigneter Alkohole sind Ethanol, Propanol, Isopropanol etc.

Weiter enthalten die erfindungsgemäßen Haarbehandlungsmittel übliche kosmetische Hilfsstoffe, beispielsweise Weichmacher, wie Glycerin und Glykol; Emollienzien; Parfüms; UV-Absorber; Farbstoffe; antistatische Mittel; Mittel zur Verbesserung der Kämmbarkeit; Konservierungsmittel; und Entschäumer.

Wenn die erfindungsgemäßen Mittel als Haarspray formuliert sind, enthalten sie eine ausreichende Menge eines Treibmittels, beispielsweise einen niedrigsiedenden Kohlenwasserstoff oder Ether, wie Propan, Butan, Isobutan oder Dimethylether. Als Treibmittel sind auch komprimierte Gase brauchbar, wie Stickstoff, Luft oder Kohlendioxid. Die Menge an Treibmittel kann dabei gering gehalten werden, um den VOC-Gehalt nicht unnötig zu erhöhen. Sie beträgt dann im Allgemeinen nicht mehr als 55 Gew.-%, bezogen auf das Gesamtgewicht des Mittels. Gewünschtenfalls sind aber auch höhere VOC-Gehalte von 85 Gew.-% und darüber möglich.

Die zuvor beschriebenen Polyurethane können auch in Kombination mit anderen Haarpolymeren in den Mitteln zur Anwendung kommen. Solche Polymere sind insbesondere:
- nicht-ionische, wasserlösliche bzw. wasserdispergierbare Polymere oder Oligomere, wie Polyvinylcaprolactam, z. B. Luviskol Plus (BASF), oder Polyvinylpyrrolidon und deren Copolymere, insbesondere mit Vinylestern, wie vinylacetat, z. B. Luviskol VA 37 (BASF); Polyamide, z. B. auf Basis von Itaconsäure und aliphatischen Diaminen; Polyvinylalkohol;
- amphotere oder zwitterionische Polymere, wie die unter den Bezeichnungen Amphomer® (Delft National) erhältlichen Octylacrylamid/Methylmethacrylat/tert.-Butylaminoethylmethacrylat/2-Hydroxypropylmethacrylat-Copolymere sowie zwitterionische Polymere, wie sie beispielsweise in den deutschen Patentanmeldungen DE 39 29 973, DE 21 50 557, DE 28 17 369 und DE 37 08 451 offenbart sind. Acrylamidopropyltrimethylammoniumchlorid/Acrylsäure- bzw. -Methacrylsäure-Copolymerisate und deren Alkali- und Ammoniumsalze sind bevorzugte zwitterionische Polymere. Weiterhin geeignete zwitterionische Polymere sind Methacroylethylbetain/Methacrylat-Copolymere, die unter der Bezeichnung Amersette® (AMERCHOL) im Handel erhältlich sind, und Copolymere aus Hydroxyethylmethacrylat, Methylmethacrylat, N,N-Dimethylaminoethylmethacrylat und Acrylsäure (Jordapon®);
- anionische Polymere, wie Vinylacetat/Crotonsäure-Copolymere, wie sie beispielsweise unter den Bezeichnungen Resyn® (NATIONAL STARCH), Luviset® (BASF) und Gafset® (GAF) im Handel sind, vinylpyrrolidon/Vinylacrylat-Copolymere, erhältlich beispielsweise unter dem Warenzeichen Luviflex® (BASF). Ein bevorzugtes Polymer ist das unter der Bezeichnung Luviflex® VBM-35 (BASF) erhältliche Vinylpyrrolidon/Acrylat-Terpolymer, Acrylsäure/Ethylacrylat/N-tert.Butylacrylamid-Terpolymere, die beispielsweise unter der Bezeichnung Ultrahold® strong (BASF) vertrieben werden, sowie Luvimer® (BASF, Terpolymer aus t-Butylacrylat, Ethylacrylat und Methacrylsäure), oder
- kationische (quaternisierte) Polymere, z. B. kationische Polyacrylatcopolymere auf Basis von N-Vinyllactamen und deren Derivaten (N-Vinylpyrrolidon, N-Vinylcaprolactam etc.) sowie übliche kationische Haarconditionerpolymere, z. B. Luviquat® (Copolymer aus Vinylpyrrolidon und Vinylimidazoliummethochlorid), Luviquat® Hold (Copolymerisat aus quaternisiertem N-Vinylimidazol, N-Vinylpyrrolidon und N-Vinylcaprolactam), Merquat® (Polymer auf Basis von Dimethyldiallylammoniumchlorid), Gafquat® (quaternäre Polymere, die durch Reaktion von Polyvinylpyrrolidon mit quaternären Ammoniumverbindungen entstehen), Polymer JR (Hydroxyethylcellulose mit kationischen Gruppen), Polyquaternium-Typen (CTFA-Bezeichnungen) etc.;
- nichtionische, siloxanhaltige, wasserlösliche oder -dispergierbare Polymere, z. B. Polyethersiloxane, wie Tegopren® (Fa. Goldschmidt) oder Belsil® (Fa. Wacker);
- Haarpolymere auf Naturbasis, wie Chitosan, Casein, Cellulosederivate, etc.

Die erfindungsgemäßen bzw. erfindungsgemäß eingesetzten Polymere können als Mischung mit einem anderen amidgruppenhaltigen Haarpolymer eingesetzt werden. Dazu zählen z. B. die in der DE-A-42 25 045 beschriebenen Polyurethane, die zuvor beschriebenen Vinylpyrrolidon/Acrylat-Terpolymere und Acrylsäure/Ethylacrylat/N-tert.-Butylacrylamid-Terpolymere (z. B. Ultrahold®strong der BASF AG), die zuvor beschriebenen amidgruppenhaltigen amphoteren Polymere (z. B. Amphomer®) und insbesondere Copolymerisate, die einen Anteil an amidgruppenhaltigen Monomeren, wie N-Vinyllactamen, von mindestens 30 Gew.-% aufweisen (z. B. Luviskol®plus und Luviskol®VA37 der BASF AG).

Die erfindungsgemäßen bzw. erfindungsgemäß eingesetzten Polymere können auch als Mischung mit einem anderen, siloxangruppenhaltigen Haarpolymer eingesetzt werden, vorzugsweise siloxangruppenhaltigen Polyurethanen.

Die anderen Haarpolymere sind vorzugsweise in Mengen bis zu 10 Gew.-%, bezogen auf das Gesamtgewicht des Mittels enthalten.

Ein bevorzugtes Haarbehandlungsmittel enthält:
a) 0,5 bis 20 Gew.-% mindestens eines, in Wasser löslichen oder dispergierbaren, erfindungsgemäßen bzw. erfindungsgemäß eingesetzten Polymers,
b) 30 bis 99,5 Gew.-%, bevorzugt 40 bis 99 Gew.-%, eines Lösungsmittels, ausgewählt unter Wasser und wassermischbares Lösungsmitteln, bevorzugt C₂- bis C₅-Alkoholen, insbesondere Ethanol, und Mischungen davon,
c) 0 bis 70 Gew.-% eines Treibmittels, vorzugsweise Dimethylether,
d) 0 bis 10 Gew.-% mindestens eines von a) verschiedenen, in Wasser löslichen oder dispergierbaren Haarpolymers,
e) 0 bis 0,3 Gew.-% mindestens eines wasserunlöslichen Silicons,
f) 0 bis 1 Gew.-% mindestens eines nichtionischen, siloxanhaltigen, in Wasser löslichen oder dispergierbaren Polymers,
sowie übliche Zusatzstoffe.

Das erfindungsgemäße Mittel kann als Komponente d) mindestens ein anderes, in Wasser lösliches oder dispergierbares Haarpolymer enthalten. Der Anteil dieser Komponente beträgt dann im Allgemeinen etwa 0,1 bis 10 Gew.-%, bezogen auf das Gesamtgewicht des Mittels. Bevorzugt können dabei wasserlösliche oder wasserdispergierbare Polyurethane eingesetzt werden, die Siloxangruppen einpolymerisiert enthalten.

Das erfindungsgemäße Mittel kann als Komponente e) mindestens ein wasserunlösliches Silicon, insbesondere ein Polydimethylsiloxan, z. B. die Abil®-Typen der Fa. Goldschmidt, enthalten. Der Anteil dieser Komponente beträgt dann im Allgemeinen etwa 0,0001 bis 0,2 Gew.-%, bevorzugt 0,001 bis 0,1 Gew.-%, bezogen auf das Gesamtgewicht des Mittels.

Das erfindungsgemäße Mittel kann als Komponente f) mindestens ein nichtionisches, siloxanhaltiges, wasserlösliches oder -dispergierbares Polymer, insbesondere ausgewählt unter den zuvor beschriebenen Polyethersiloxanen, enthalten. Der Anteil dieser Komponente beträgt dann im Allgemeinen etwa 0,001 bis 2 Gew.-%, bezogen auf das Gesamtgewicht des Mittels.

Das erfindungsgemäße Mittel kann zusätzlich gegebenenfalls einen Entschäumer, z. B. auf Silicon-Basis, enthalten. Die Menge des Entschäumers beträgt im Allgemeinen bis zu etwa 0,001 Gew.-%, bezogen auf die Gesamtmenge des Mittels.

Gegenstand der Erfindung ist auch ein Haarbehandlungsmittel, enthaltend:
a) 0,5 bis 20 Gew.-% mindestens eines in Wasser löslichen oder dispergierbaren Polymers, das wenigstens ein siloxangruppenfreies Urethan(meth)acrylat und wenigstens ein radikalisch polymerisierbares, α,β-ethylenisch ungesättigtes Monomer M) einpolymerisiert enthält, wobei das siloxangruppenfreie Urethan(meth)acrylat die Komponenten a), b) und c), wie in Anspruch 1 definiert und gegebenenfalls wenigstens eine weitere Komponente, die ausgewählt ist unter den Komponenten e), f), und g), wie in Anspruch 2 definiert, eingebaut enthält,
b) 30 bis 99,5 Gew.-%, bevorzugt 40 bis 99 Gew.-%, wenigstens eines Lösungsmittels, ausgewählt unter wasser, wassermischbaren Lösungsmittel und Mischungen davon,
c) 0 bis 70 Gew.-% eines Treibmittels,
d) 0 bis 10 Gew.-% mindestens eines von a) verschiedenen, in Wasser löslichen oder dispergierbaren Haarpolymers,
e) 0 bis 0,3 Gew.-% mindestens eines wasserunlöslichen Silicons,
f) 0 bis 1 Gew.-% mindestens eines nichtionischen, siloxanhaltigen, in Wasser löslichen oder dispergierbaren Polymers.

Geeignete Polymere a), die wenigstens ein siloxangruppenfreies Urethan(meth)acrylaten und wenigstens ein radikalisch polymerisierbares, α,β-ethylenisch ungesättigtes Monomer M) einpolymerisiert enthalten und Verfahren zu ihrer Herstellung sind die bereits zuvor beschriebenen. Geeignete Komponenten b), c), d), e) und f) sind ebenfalls die zuvor für die erfindungsgemässen Mittel auf Basis siloxangruppenhaltiger Polymere genannten. Auf die Ausführungen zu den erfindungsgemässen Mitteln auf Basis siloxangruppenhaltiger Polymere wird vollständig Bezug genommen.

Die erfindungsgemäßen Mittel besitzen den Vorteil, dass sie einerseits den Haaren die gewünschte Festigkeit verleihen und andererseits die Polymere leicht auswaschbar (redispergierbar) sind. Darüber hinaus lassen sich Haarbehandlungsmittel mit einem in VOC-Gehalt von weniger als 85 Gew.-%, bevorzugt weniger als 60 Gew.-%, und auch rein wässrige Formulierungen herstellen, selbst wenn sie als Haarspray formuliert sind.

Die Erfindung wird anhand der folgenden, nicht einschränkenden Beispiele näher erläutert.

### Beispiele

### Urethan(meth)acrylatherstellung

Zur Herstellung der Urethan(meth)acrylate der folgenden Tabelle 1, die hydroxylgruppenhaltige Komponenten eingebaut enthalten (Beispiel 2: Neopentylglykol, Beispiel 4: Dimethylolpropansäure) wurde in einem Vierhalskolben, der mit Rührer, Tropftrichter, Thermometer, Rückflusskühler und einer Vorrichtung für das Arbeiten unter Stickstoff ausgestattet war, die hydroxylgruppenhaltige Komponente in einer Menge nach Tabelle 1 in Aceton (Feststoffgehalt der resultierenden Reaktionslösung ca. 80%) vorgelegt und unter Rühren auf eine Temperatur von 60 °C erhitzt. Anschließend wurde unter Rühren Isophorondiisocyanat in einer Menge nach Tabelle 1 zugetropft, wobei die Reaktionstemperatur anstieg. Bei Rückfluss wurde das Reaktionsgemisch dann so lange gerührt, bis der Isocyanatgruppengehalt des Gemisches praktisch konstant blieb und anschließend unter Rühren auf Raumtemperatur abgekühlt. Dann gab man bei einer Temperatur von etwa 30 °C ein Polysiloxandiamin (Mₙ = 900 g/mol, Tegomer® A Si 2122 der Fa. Goldschmidt) und tert.-Butylaminoethylmethacrylat (in Form einer 50 gew.-%-igen Lösung in Aceton) in einer Menge nach Tabelle 1 zu den wie zuvor beschrieben hergestellten Polyurethanpräpolymeren. Die Mischung wurde dann noch weitere 20 Minuten bei etwa 50 °C gerührt und anschließend ein Polyethylenglykoldiamin (O,O'-Bis(2-aminopropyl)polyethylenglykol 800, Mₙ = 900 g/mol der Fa. Fluka, in Form einer 70 %-igen Lösung in Ethanol) zugesetzt. Anschließend wurde das Reaktionsgemisch weitere 30 Minuten bei 30 °C gerührt.

Zur Herstellung der Urethan(meth)acrylate, die keine hydroxylgruppenhaltigen Komponenten eingebaut enthalten (Beispiele 1, 3 und 5) wurde ein Gemisch aus einem Polysiloxandiamin (Beispiele 1 und 3: Mₙ = 900 g/mol; Beispiel 5: Mₙ = 2800 g/mol), tert.-Butylaminoethylmethacrylat (in Form einer 50 gew.-%-igen Lösung in Aceton) und gegegebenenfalls N-Methyldipropylentriamin (Beispiel 3) in einer Menge nach Tabelle 1, in der zuvor beschriebenen Apparatur, vorgelegt und unter Rühren auf etwa 30 °C erhitzt. Dann wurde unter Rühren Isophorondiisocyanat in einer Menge nach Tabelle 1 zugetropft, und anschließend das Reaktionsgemisch bei einer Temperatur von etwa 30 °C so lange gerührt, bis der Isocyanatgruppengehalt praktisch konstant blieb. Dann wurde ein Polyethylenglykoldiamin ebenfalls in einer Menge nach Tabelle 1 bei Raumtemperatur zugegeben und das Reaktionsgemisch noch weitere 30 Minuten bei etwa 30 °C gerührt. Abschließend wurde das Reaktionsgemisch in allen Fällen mit Ethanol auf 50 Gew.-% verdünnt.

**Tabelle 1**

| Bsp. Nr. | Polysiloxandiamin I¹⁾ [mol] | Polysiloxandiamin II²⁾ [mol] | PEGDA³⁾ [mol] | NPG⁴ [mol] | MADPTA⁵⁾ [mol] | DMPA⁶) [mol] | tBAEMA⁷⁾ [mol] | IDPI⁸) [mol] |
|---|---|---|---|---|---|---|---|---|
| 1 | 1 | - | 4,5 | - | - | - | 1 | 6 |
| 2 | 1 | - | 3,5 | 1 | - | - | 1 | 6 |
| 3 | 1 | - | 3,5 | - | 1 | - | 1 | 6 |
| 4 | 1 | - | 3,5 | - | - | 1 | 1 | 6 |
| 5 | - | 0,5 | 5 | - | - | - | 1 | 6 |

| | | | | | | | | |
|---|---|---|---|---|---|---|---|---|
| 1) Polysiloxandiamin, Mₙ = 900 g/mol (Tegomer® A-Si 2122 der Fa. Goldschmidt) | | | | | | | | |
| 2) Polysiloxandiamin, Mₙ = 2800 g/mol (Tegomer ® A-Si 2322 der Fa. Goldschmidt) | | | | | | | | |
| 3) O,O'-Bis(2-aminopropyl)polyethylenglykol 800, Mₙ = 900 g/mol (Fa. Fluka) | | | | | | | | |
| 4) Neopentylglykol | | | | | | | | |
| 5) N-Methyldipropylentriamin | | | | | | | | |
| 6) Dimethylolpropansäure | | | | | | | | |
| 7) tert.-Butylaminoethylmethacrylat | | | | | | | | |
| 8) Isophorondiisocyanat | | | | | | | | |

### Beispiel 6

Urethan(meth)acrylatherstellung in Ethanol

Zur Herstellung eines Urethan(meth)acrylates mit Harnstoffgruppen wurde in einem Vierhalskolben, der mit Rührer, Tropftrichter, Thermometer, Rückflusskühler und einer Vorrichtung für das Arbeiten unter Stickstoff ausgestattet war, ein Gemisch aus einem Polysiloxanamin (Mₙ = 2000 g/mol, Aminzahl ca. 28 (entsprechend 0,04 Mol Aminogruppen), MAN® 00078 der Fa. Hüls) und 80 g Ethanol vorgelegt. Bei einer Temperatur von etwa 20°C wurden unter Rühren 133 g (ca. 0,6 mol) Isophorondiisocyanat zugetropft und das Reaktionsgemisch anschliessend noch weitere 20 Minuten bei Umgebungstemperatur gerührt. Dann gab man 7,4 g (0,04 mol) tert.-Butylaminoethylmethacrylat gelöst in 100 g Ethanol und anschliessend ein Gemisch aus 315 g (0,35 mol) O,O'-Bis(2-aminopropyl)polyethylenglykol 800 (Mₙ = 900 g/mol der Fa. Fluka) und 33,5 g (0,23 mol) N-Methyldipropylentriamin gelöst in 400 g Ethanol zu. Dabei wurde eine Reaktionstemperatur von höchstens 40°C eingehalten. Das Reaktionsgemisch wurde noch weitere 30 Minuten bei etwa 40°C gerührt und abschliessend filtriert. Es wurde eine klare, farblose 30%ige ethanolische Lösung eines harnstoffgruppenhaltigen Produkts erhalten.

Analog der Reaktionsvorschrift aus Beispiel 6 können die Urethan(meth)acrylate mit Harnstoffgruppen der zuvor beschriebenen Beispiele 1, 3 und 5 hergestellt werden.

### Beispiele 7 bis 29

| | | |
|---|---|---|
| Zulauf 1: | 220 g | Monomerengemisch nach Tabelle 2 |
| Zulauf 2: | 0,5 g | tert.-Butylperpivalat |
| | 100 g | Ethanol |
| Zulauf 3: | 1,5 g | tert.-Butylperpivalat |
| | 82 g | Ethanol |

In einer Rührapparatur mit Rückflusskühler und zwei separaten Zulaufvorrichtungen wurden unter Stickstoffatmosphäre 44 g Zulauf 1 und 12 g Zulauf 2 in 120 g Ethanol vorgelegt und unter Rühren auf etwa 80 °C erwärmt. Nach dem Anpolymerisieren, erkennbar an einer beginnenden Viskositätserhöhung, wurde der Rest von Zulauf 1 innerhalb von 4 Stunden und der Rest von Zulauf 2 innerhalb von 5 Stunden zugegeben, wobei die Innentemperatur auf etwa 70 bis 80 °C gehalten wurde. Anschließend ließ man noch 2 Stunden bei einer Temperatur von etwa 75 bis 80 °C nachreagieren und gab dann Zulauf 3 innerhalb von 2 Stunden zu. Nach dem Ende der Zugabe wurde noch ca. 4 Stunden bei dieser Temperatur nachpolymerisiert. Anschließend wurden die säuregruppenhaltigen Polymerisate mit einer Base nach Tabelle 2 und die amingruppenhaltigen Polymerisate mit einer Säure neutralisiert. Die K-Werte und Neutralisationsgrade der Polymere sind ebenfalls in Tabelle 2 angegeben.

polymere auf Basis nichtionischer Komponenten oder auf Basis von Urethan(meth)acrylaten, die bereits neutralisierte bzw. quaternisierte Gruppen tragen, können im Allgemeinen direkt zur Formulierung von Haarpräparaten eingesetzt werden.

**Tabelle 2**

| Bsp. Nr. | Urethan(meth)acrylat aus Bsp. Nr. [Gew.-%) | | | TBA¹) [Gew.-%] | MAS²) [Gew.-%] | DMAPMA³) [Gew.-%] | VP⁴) [Gew.-%l | VCap⁵) [Gew.-%] | Neutralisationsmittel (-grad) | K-Wert⁷) |
|---|---|---|---|---|---|---|---|---|---|---|
| 7 | (1) | | | 65 | 251 | - | - | - | AMP⁶) | 43,7 |
| | 10 | | | | | | | | (95%) | |
| 8 | (1) | | | 60 | 20 | - | - | - | AMP | 41,9 |
| | 20 | | | | | | | | (95%) | |
| 9 | (1) | | | 52 | 18 | - | - | - | AMP | 38,9 |
| | 30 | | | | | | | | (95%) | |
| 10 | (2) | | | 65 | 25 | - | - | - | AMP | 45,3 |
| | 10 | | | | | | | | (100%) | |
| 11 | (3) | | | 65 | 25 | - | - | - | AMP | 46,0 |
| | 10 | | | | | | | | (100%) | |
| 12 | (4) | | | 65 | 25 | - | - | - | AMP (95%) | 40,1 |
| | 10 | | | | | | | | | |
| 13 | (5) | | | 65 | 25 | - | - | - | AMP (100%) | 42,8 |
| | 10 | | | | | | | | | |
| 14 | (4) | | | 55 | 20 | - | - | - | AMP | 39,6 |
| | 25 | | | | | | | | (100%) | |
| 15 | (1) | | | 55 | 25 | 10 | - | - | AMP | 38,3 |
| | 10 | | | | | | | | (100%) | |
| 16 | (1) | | | 40 | - | 15 | 35 | - | H₃PO₄ | 37,9 |
| | 10 | | | | | | | | (50%) | |
| 17 | (1) | | | - | - | 10 | 35 | 35 | H₃PO₄ | 45,2 |
| | 20 | | | | | | | | (50%) | |
| 18 | (2) | | | - | - | 10 | 35 | 25 | H₃PO₄ | 41,0 |
| | 30 | | | | | | | | (50%) | |
| 19 | (3) | | | - | - | - | 35 | 35 | H₃PO₄ | 39,0 |
| | 30 | | | | | | | | (30%) | |
| 20 | (3) | | | - | - | - | - | 70 | H₃PO₄ | 42,0 |
| | 30 | | | | | | | | (30%) | |

| Bsp. Nr. | Urethan(meth)acrylat aus Bsp. Nr. [Gew.-%) | TBA¹) [Gew.-%] | n-BA⁸⁾ | NTBAM⁹⁾ | MAS²) [Gew.-%] | DMAPMA³) [Gew.-%] | VP⁴) [Gew.-%l | VCap⁵) [Gew.-%] | Neutralisationsmittel (-grad) | K-Wert⁷) |
|---|---|---|---|---|---|---|---|---|---|---|
| 21 | (1) | 30 | 35 | - | 25 | - | - | - | AMP⁶⁾ (95%) | 41,3 |
| | | | | | | | | | | |
| 22 | (1) | - | 45 | 20 | 25 | - | - | - | AMP | 43,7 |
| | | | | | | | | | (95%) | |
| 23 | (6) | 22 | 45 | - | 23 | - | - | - | AMP (95%) | 44,1 |
| | 10 | | | | | | | | | |
| 24 | (6) | - | 55 | 12 | 23 | - | - | - | AMP | 45,0 |
| | 10 | | | | | | | | (95%) | |
| 25 | (1) | 20 | 20 | - | - | 15 | - | 35 | H₃PO₄ | 38,9 |
| | 10 | | | | | | | | (50%) | |
| 26 | (1) | - | 20 | 20 | - | 12 | - | 38 | H₃PO₄ | 37,5 |
| | 10 | | | | | | | | (50%) | |
| 27 | (6) | 40 | - | - | - | 15 | - | 35 | H₃PO₄ | 40,2 40,2 |
| | 10 | | | | | | | | (50%) | |
| 28 | (6) | 20 | 20 | - | - | 12 | - | 38 | H₃PO₄ | 42,1 |
| | 10 | | | | | | | | (50%) | |
| 29 | (6) | - | 20 | 20 | - | 15 | - | 35 | H₃PO₄ | 43,0 |
| | 10 | | | | | | | | (50%) | |

| | | | | | | | | | | |
|---|---|---|---|---|---|---|---|---|---|---|
| 1) tert.-Butylacrylat | | | | | | | | | | |
| 2) N,N-Dimethylaminopropylmethacrylamid | | | | | | | | | | |
| 3) Methacrylsäure | | | | | | | | | | |
| 4) Vinylpyrrolidon | | | | | | | | | | |
| 5) Vinylcaprolactam | | | | | | | | | | |
| 6) 2-Amino-2-methylpropanol | | | | | | | | | | |
| 7) gemessen als 1 %-ige Lösung in Ethanol | | | | | | | | | | |
| 8) n-Butylacrylat | | | | | | | | | | |
| 9) N-(tert.-Butyl)acrylamid | | | | | | | | | | |

### Anwendungstechnische Beispiele

### Beispiele 30 bis 52

### Aerosol-Haarspray-Formulierungen mit einem VOC-Gehalt von 97 Gew.-%:

| | |
|---|---|
| Polymer gemäß Beispiel 7-29 | 3,00 Gew.-% |
| Ethanol | 62,00 Gew.-% |
| Dimethylether | 34,96 Gew.-% |
| Parfüm, Zusatzstoffe | q.s. |

### Beispiele 53 bis 75

### Kompakte Aerosol-Haarspray-Formulierungen mit einem VOC-Gehalt von 90 Gew.-%:

| | |
|---|---|
| Polymer gemäß Beispiel 7-29 | 10,00 Gew.-% |
| Ethanol | 55,00 Gew.-% |
| Dimethylether | 34,96 Gew.-% |
| Parfüm, Zusatzstoffe | q.s. |

### Beispiele 76 bis 98

### Haarspray-Formulierungen mit einem VOC-Gehalt von 80 Gew.-%:

| | |
|---|---|
| Polymer gemäß Beispiel 7-29 | 5,00 Gew.-% |
| Ethanol | 45,00 Gew.-% |
| Wasser | 15,00 Gew.-% |
| Dimethylether | 34,96 Gew.-% |
| Parfüm, Zusatzstoffe | q.s. |

### Beispiele 99 bis 121

### Haarspray-Formulierungen mit einem VOC-Gehalt von 55 Gew.-%:

| | |
|---|---|
| Polmer gemäß Beispiel 7-29 | 3,00 Gew.-% |
| Ethanol | 20,00 Gew.-% |
| Wasser | 42,00 Gew.-% |
| Dimethylether | 34,96 Gew.-% |
| Parfüm, Zusatzstoffe | q.s. |

### Beispiele 122 bis 137

### Pump-Haarspray-Formulierungen mit einem VOC-Gehalt 55 Gew.-%:

| | |
|---|---|
| Polymer gemäß Beispiel 8, 9, 14, 17-29 | 10,00 Gew.-% |
| Wasser | 37,00 Gew.-% |
| Ethanol | 55,00 Gew.-% |
| Parfüm, Zusatzstoffe | q.s. |

### Beispiele 138 bis 160

| Schaum-Conditioner | :[Gew.-%] |
|---|---|
| Polymer 7-29 (25%ige wässrige Lösung | 20,00 |
| Cremophor® A¹⁰⁾ | 0,20 |
| Comperlan® KD¹¹⁾ | 0,10 |
| Wasser | 69,70 |
| Propan/Butan | 9,96 |
| Parfüm, Konservierungsmittel | q.s. |

| | |
|---|---|
| ¹⁰⁾ CTFA-Name: Ceteareth 25, Fa. BASF AG, Umsetzungsprodukt aus Fettalkohol und Ethylenoxid | |
| ¹¹⁾ CTFA-Name: Coamide DEA, Fa. Henkel, Kokosfettsäureamid | |

Zur Herstellung der Schaum-Conditioner werden die Komponenten eingewogen und unter Rühren gelöst. Anschließend werden sie in einen Spender abgefüllt und das Treibgas zugesetzt.

### Beispiele 161 bis 183

| Conditioner-Shampoo: | | [Gew.-%] |
|---|---|---|
| A) | Texapon® NSO 28%ig¹²⁾ | 50,00 |
| | Comperlan® KD | 1,00 |
| | Polymer 1-14 (25%ige wässrige Lösung | 20,00 |
| | Parfümöl | q.s. |
| B) | Wasser | 27,5 |
| | Natriumchlorid | 1,5 |
| | Konservierungsmittel | q.s. |

| | | |
|---|---|---|
| ¹²⁾ Natriumlaurylsulfat, Fa. Henkel | | |

Zur Herstellung der Conditioner-Shampoos werden die Komponenten A) und B) getrennt eingewogen und unter Mischen gelöst. Dann wird Phase B) langsam unter Rühren zu Phase A) gegeben.

### Beispiele für Anwendungen in der Hautkosmetik

### Beispiele 184 bis 206

**O/W-Cremes**

| Ölphase: | Gew.-% | CTFA-Name: |
|---|---|---|
| Cremophor® A6 (BASF AG) | 3,5 | Ceteareth-6 (Stearylalkohol-Ethoxylat) |
| Cremophor® A25 (BASF AG) | 3,5 | Ceteareth-25 (Fettalkohol-Ethoxylat) |
| Glycerinmonostearat s.e. | 2,5 | Glycerylstearate |
| Paraffinöl | 7,5 | |
| Cetylalkohol | 2,5 | |
| Luvitol® EHO (BASF AG) | 3,2 | Cetearyloctanoat |
| Vitamin-E-acetate | 1,0 | Tocopherylacetat |
| Nip-Nip®, Nipa Laboratories Ltd., USA | 0,1 | Methyl- und Propyl-4-hydroxybenzoate (7:3) |

| Wasserphase: | Gew.-% | |
|---|---|---|
| Polymer 7-29 | 1,5 | |
| Wasser | 73,6 | |
| 1,2-Propylenglykol | 1,0 | Propylenglykol |
| Germall II, Sutton Laboratories Inc., USA | 0,1 | Imidazolidinylharnstoff |

Zur Herstellung der Cremes werden die Komponenten für Öl- und Wasserphase getrennt eingewogen und bei 80 °C homogenisiert. Dann gibt man die Wasserphase langsam unter Rühren zu der Ölphase. Anschließend lässt man unter Rühren auf Raumtemperatur abkühlen.

### Beispiele 207 bis 229

**O/W-Lotions**

| Ölphase: | Gew.-% | CTFA-Name: |
|---|---|---|
| Cremophor® A6 (BASF AG) | 2,0 | Ceteareth-6 (Stearylalkohol-Ethoxylat) |
| Cremophor® A25 (BASF AG) | 2,0 | Ceteareth-25 (Fettalkohol-Ethoxylat) |
| Glycerinmonostearat s.e. | 6,0 | Glycerylstearate |
| Paraffinöl | 0,9 | Paraffinöl |
| Tegiloxan® 100 | 0,1 | Dimethicone (Polydimethylsiloxan) |
| Cetylalkohol | 1,5 | Cetylalkohol |
| Luvitol® EHO (BASF AG) | 12,0 | Cetearyloctanoat |
| Vitamin-E-acetate | 0,4 | Tocopherylacetat |
| Nip-Nip®, Nipa Laboratories Ltd., USA | 0,1 | Methyl- und Propyl-4-hydroxybenzoate (7:3) |

| Wasserphase: | Gew.-% | |
|---|---|---|
| Polymer 7-29 | 1,0 | |
| wasser | 73,4 | |
| 1,2-Propylenglykol | 1,0 | Propylenglykol |
| Germall II, Sutton Laboratories Inc., USA | 0,1 | Imidazolidinylharnstoff |

Zur Herstellung der O/W-Lotionen werden die Komponenten für Öl- und Wasserphase getrennt eingewogen und bei 80 °C homogenisiert. Dann gibt man die Wasserphase langsam unter Rühren zu der Ölphase. Anschließend lässt man unter Rühren auf Raumtemperatur abkühlen.

## Patentansprüche

1. Haarbehandlungsmittel, das wenigstens ein wasserlösliches oder wasserdispergierbares Polymer sowie übliche kosmetische Hilfsstoffe enthält, wobei das Polymer wenigstens ein Urethan(meth)acrylat und wenigstens ein radikalisch polymerisierbares, α,β-ethylenisch ungesättigtes Monomer M) einpolymerisiert enthält, wobei das Urethan(meth)acrylat ausgewählt ist unter radikalisch polymerisierbaren, siloxangruppenhaltigen Urethan(meth)acrylaten, die
a) wenigstens eine Verbindung, die mindestens ein aktives Wasserstoffatom und mindestens eine radikalisch polymerisierbare, α,β-ethylenisch ungesättigte Doppelbindung pro Molekül enthält,
b) wenigstens ein Diisocyanat,
c) wenigstens eine Verbindung, die zwei aktive Wasserstoffatome pro Molekül enthält,
d) wenigstens eine Verbindung, die mindestens ein aktives Wasserstoffatom und mindestens eine Siloxangruppe pro Molekül enthält,
eingebaut enthalten, und den Salzen davon.

2. Mittel nach Anspruch 1, wobei die Urethan(meth)acrylate zusätzlich wenigstens eine Komponente, die ausgewählt ist unter
e) Verbindungen, die zwei oder mehrere aktive Wasserstoffatome und mindestens eine ionogene und/oder ionische Gruppe pro Molekül enthalten,
f) einwertigen Alkoholen, Aminen mit einer primären oder sekundären Aminogruppe, aliphatischen, cycloaliphatischen oder aromatischen Monoisocyanaten und Mischungen davon,
g) α,β-ethylenisch ungesättigten Verbindungen, die zusätzlich wenigstens eine Isocyanatgruppe pro Molekül enthalten,
und Mischungen davon, eingebaut enthalten.

3. Mittel nach einem der Ansprüche 1 oder 2, wobei die Komponente d) ausgewählt ist unter:
- Polysiloxanen der allgemeinen Formel I.1 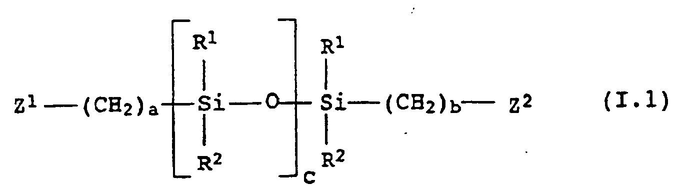 worin
a und b unabhängig voneinander für 2 bis 8 stehen,
c für 3 bis 100 steht,
R¹ und R² unabhängig voneinander für C₁- bis C₈-Alkyl, Benzyl oder Phenyl stehen,
Z¹ und Z² unabhängig voneinander für OH, NHR³ oder einen Rest der Formel II
-O-(CH₂CH₂O)ᵥ(CH₂CH(CH₃)O)_{w}-H (II)
stehen, wobei
in der Formel II die Reihenfolge der Alkylenoxideinheiten beliebig ist und
v und w unabhängig voneinander für eine ganze Zahl von 0 bis 200 stehen, wobei die Summe aus v und w > 0 ist,
R³ für Wasserstoff, C₁- bis C₈-Alkyl oder C₅- bis C₈₋Cycloalkyl steht;
- Polysiloxanen der allgemeinen Formel I.2 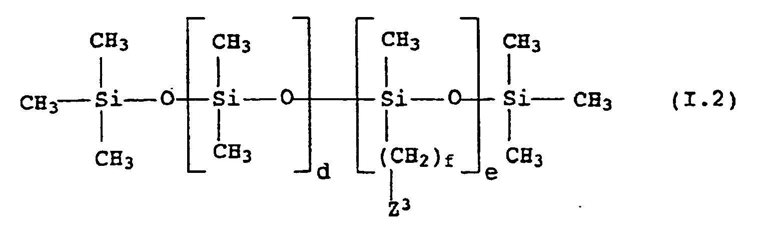 worin
die Reihenfolge der Siloxaneinheiten beliebig ist,
d und e unabhängig voneinander für 0 bis 100 stehen, wobei die Summe aus d und e mindestens 2 ist,
f für eine ganze Zahl von 2 bis 8 steht,
Z³ für OH, NHR³ oder einen Rest der Formel II steht,
wobei R³ für Wasserstoff, C₁- bis C₈-Alkyl, C₅- bis C₈₋Cycloalkyl oder einen Rest der Formel -(CH₂)ᵤ-NH₂ steht, wobei u für eine ganze Zahl von 1 bis 10 steht,
- Polysiloxanen mit sich wiederholenden Einheiten der allgemeinen Formel I.3 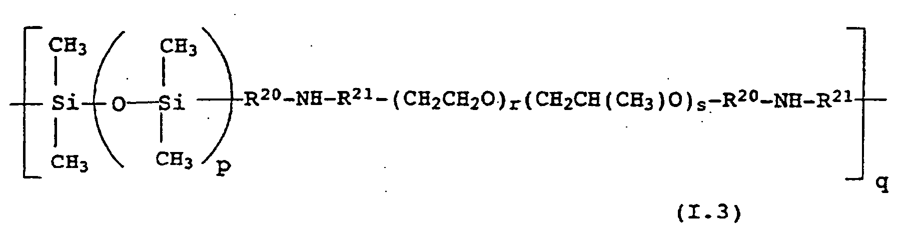 worin
p für eine ganze Zahl von 0 bis 100 steht,
q für eine ganze Zahl von 1 bis 8 steht,
R²⁰ und R²¹ unabhängig voneinander für C₁- bis C₈-Alkylen stehen,
die Reihenfolge der Alkylenoxideinheiten beliebig ist und
r und s unabhängig voneinander für eine ganze Zahl von 0 bis 200 stehen, wobei die Summe aus r und s > 0 ist,
- Polysiloxanen der allgemeinen Formel I.4 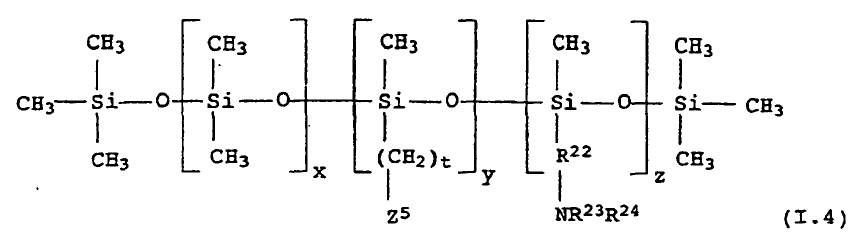 worin
R²² für einen C₁- bis C₈-Alkylenrest steht,
R²³ und R²⁴ unabhängig voneinander für Wasserstoff, C₁- bis C₈-Alkyl oder C₅- bis C₈-Cycloalkyl stehen,
die Reihenfolge der Siloxaneinheiten beliebig ist,
x, y und z unabhängig voneinander für 0 bis 100 stehen, wobei die Summe aus x, y und z mindestens 3 ist,
t für eine ganze Zahl von 2 bis 8 steht,
Z⁵ für einen Rest der Formel VII
-(OCH₂CH₂)ᵢ(OCH₂CH(CH₃))ⱼ-R²⁵ (VII)
steht, worin
die Reihenfolge der Alkylenoxideinheiten beliebig ist und i und j unabhängig voneinander für eine ganze Zahl von 0 bis 200 stehen, wobei die Summe aus i und j > o ist,
R²⁵ für Wasserstoff oder einen C₁- bis C₈-Alkylrest steht,
und Mischungen davon.

4. Mittel nach einem der vorhergehenden Ansprüche, wobei das Monomer M) ausgewählt ist unter
M1) im Wesentlichen hydrophoben, nichtionischen Verbindungen, bevorzugt Estern α,β-ethylenisch ungesättigter Mono- und/oder Dicarbonsäuren mit C₁-C₃₀-Alkanolen, Amiden α,β-ethylenisch ungesättigter Mono- und/oder Dicarbonsäuren mit Mono- und Di-C₁-C₃₀-alkylaminen, Estern von Vinylalkohol und Allylalkohol mit C₁-C₃₀-Monocarbonsäuren, Vinylethern, Vinylaromaten, Vinylhalogeniden, Vinylidenhalogeniden, C₂-C₈-Monoolefinen, nichtaromatischen Kohlenwasserstoffen mit mindestens 2 konjugierten Doppelbindungen und Mischungen davon,
M2) Verbindungen mit einer radikalisch polymerisierbaren, α,β-ethylenisch ungesättigten Doppelbindung und mindestens einer ionogenen und/oder ionischen Gruppe pro Molekül,
M3) im Wesentlichen hydrophilen, nichtionischen Verbindungen, bevorzugt N-Vinylamiden, N-Vinyllactamen, primären Amiden α,β-ethylenisch ungesättigter Monocarbonsäuren, vinyl- und allylsubstituierten heteroaromatischen Verbindungen, Estern α,β-ethylenisch ungesättigter Mono- und Dicarbonsäuren mit C₁-C₃₀-Alkandiolen, Estern und Amiden α,β-ethylenisch ungesättigter Mono- und Dicarbonsäuren mit C₂-C₃₀-Aminoalkoholen, die eine primäre oder sekundäre Aminogruppe aufweisen, Polyetheracrylaten, und Mischungen davon,
und Mischungen davon.

5. Mittel nach einem der vorhergehenden Ansprüche, wobei das Polymer
- 0,05 bis 80 Gew.-%, bevorzugt 0,1 bis 50 Gew.-%, des/der Urethan (meth) acrylats(e), und
- 20 bis 99,95 Gew.-%, bevorzugt 50 bis 99,9 Gew.-%, wenigstens einer Komponente M),
einpolymerisiert enthält.

6. Mittel nach einem der vorhergehenden Ansprüche, wobei das Polymer
- 0,1 bis 50 Gew.-%, bevorzugt 0,5 bis 35 Gew.-%, des/der Urethan (meth) acrylats(e),
- 40 bis 75 Gew.-%, bevorzugt 45 bis 73 Gew.-%, wenigstens einer Komponente M1),
- 10 bis 35 Gew.-%, bevorzugt 18 bis 30 Gew.-%, wenigstens einer Komponente M2), 0 bis 30 Gew.-%, wenigstens einer Komponente M3),
einpolymerisiert enthält.

7. Mittel nach einem der vorhergehenden Ansprüche, wobei das Polymer
- 0,1 bis 50 Gew.-%, bevorzugt 0,5 bis 35 Gew.-%, des/der Urethan(meth)acrylats(e),
- 0 bis 50 Gew.-% wenigstens einer Komponente M1),
- 0 bis 20 Gew.-%, wenigstens einer Komponente M2),
- 25 bis 80 Gew.-%, wenigstens einer Komponente M3), einpolymerisiert enthält.

8. Haarbehandlungsmittel nach einem der vorhergehenden Ansprüche, enthaltend
a) 0,5 bis 20 Gew.-% mindestens eines in Wasser löslichen oder dispergierbaren Polymers,
b) 30 bis 99,5 Gew.-%, bevorzugt 40 bis 99 Gew.-%, wenigstens eines Lösungsmittels, ausgewählt unter Wasser, wassermischbaren Lösungsmittel und Mischungen davon,
c) 0 bis 70 Gew.-% eines Treibmittels,
d) 0 bis 10 Gew.-% mindestens eines von a) verschiedenen, in Wasser löslichen oder dispergierbaren Haarpolymers,
e) 0 bis 0,3 Gew.-% mindestens eines wasserunlöslichen Silicons,
f) 0 bis 1 Gew.-% mindestens eines nichtionischen, siloxanhaltigen, in Wasser löslichen oder dispergierbaren Polymers.

9. Haarbehandlungsmittel, enthaltend
a) 0,5 bis 20 Gew.-% mindestens eines in Wasser löslichen oder dispergierbaren Polymers, das wenigstens ein siloxangruppenfreies Urethan(meth)acrylat und wenigstens ein radikalisch polymerisierbares, α,β-ethylenisch ungesättigtes Monomer M) einpolymerisiert enthält, wobei das siloxangruppenfreie Urethan(meth)acrylat die Komponenten a), b) und c), wie in Anspruch 1 definiert und gegebenenfalls wenigstens eine weitere Komponente, die ausgewählt ist unter den Komponenten e), f), und g), wie in Anspruch 2 definiert, eingebaut enthält,
b) 30 bis 99,5 Gew.-%, bevorzugt 40 bis 99 Gew.-%, wenigstens eines Lösungsmittels, ausgewählt unter Wasser, wassermischbaren Lösungsmittel und Mischungen davon,
c) 0 bis 70 Gew.-% eines Treibmittels,
d) 0 bis 10 Gew.-% mindestens eines von a) verschiedenen, in Wasser löslichen oder dispergierbaren Haarpolymers,
e) 0 bis 0,3 Gew-% mindestens eines wasserunlöslichen Silicons,
f) 0 bis 1 Gew.-% mindestens eines nichtionischen, siloxanhaltigen, in Wasser löslichen, oder dispergierbaren Polymers.

10. Verwendung von wasserlöslichen oder wasserdispergierbaren polymeren, die ausgewählt sind unter Polymeren, wie in einem der Ansprüche 1 bis 7 definiert, und Mischungen davon, in der Haarkosmetik, bevorzugt als Festigerpolymer in Haarsprays, Schaumfestigern, Haarmousse, Haargel und Schampoos, und in der Hautkosmetik, bevorzugt in Cremes, pigmenthaltigen Hautkosmetika und wachshaltigen Hautkosmetika.

11. Radikalisch polymerisierbare, siloxangruppenhaltige Urethan(meth) acrylate, wie in einem der Ansprüche 1 bis 3 definiert, die als Komponente a) tert.-Butylaminoethylacrylat und/oder tert.-Butylaminoethylmethacrylat, eingebaut enthalten, und die Salze davon.

12. wasserlösliches oder wasserdispergierbares Polymer, das wenigstens ein Urethan(meth)acrylat nach Anspruch 11 und wenigstens ein radikalisch polymerisierbares, α,β-ethylenisch ungesättigtes Monomer M) einpolymerisiert enthält.

## Claims

1. A hair-treatment composition which comprises at least one water-soluble or water-dispersible polymer and also customary cosmetic auxiliaries, the polymer comprising, in copolymerized form, at least one urethane (meth)acrylate and at least one free-radically polymerizable, α,β-ethylenically unsaturated monomer M) and the urethane (meth)acrylate being chosen from free-radically polymerizable, siloxane-containing urethane (meth)acrylates which comprise, in incorporated form,
a) at least one compound which comprises at least one active hydrogen atom and at least one free-radically polymerizable α,β-ethylenically unsaturated double bond per molecule,
b) at least one diisocyanate,
c) at least one compound which comprises two active hydrogen atoms per molecule,
d) at least one compound which comprises at least one active hydrogen atom and at least one siloxane group per molecule,
and the salts thereof.

2. The composition according to claim 1, wherein the urethane (meth)acrylate additionally comprises, in incorporated form, at least one component chosen from
e) compounds which comprise two or more active hydrogen atoms and at least one ionogenic and/or ionic group per molecule,
f) monohydric alcohols, amines with a primary or secondary amino group, aliphatic, cycloaliphatic or aromatic monoisocyanates and mixtures thereof,
g) α,β-ethylenically unsaturated compounds which additionally comprise at least one isocyanate group per molecule,
and mixtures thereof.

3. The composition according to either of claims 1 and 2, wherein component d) is chosen from:
- polysiloxanes of the formula I.1 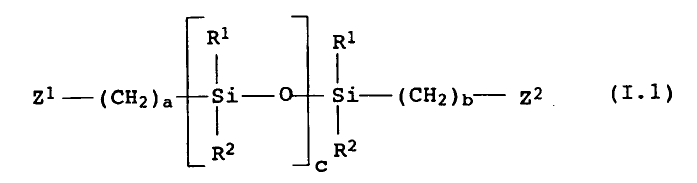 in which
a and b independently of one another are from 2 to 8,
c is from 3 to 100,
R¹ and R² independently of one another are C₁-C₈-alkyl, benzyl or phenyl,
Z¹ and Z² independently of one another are OH, NHR³ or a radical of the formula II
-O-(CH₂CH₂O)ᵥ(CH₂CH(CH₃)O_{)w}-H (II)
where
in formula II the order of the alkylene oxide units is arbitrary, and
v and w independently of one another are an integer from 0 to 200, the sum v + w being > 0,
R³ is hydrogen, C₁-C₈-alkyl or C₅-C₈-cycloalkyl;
- polysiloxanes of the formula I.2 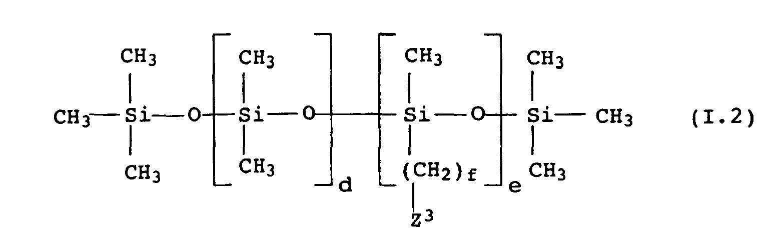 in which
the order of the siloxane units is arbitrary,
d and e independently of one another are from 0 to 100, the sum d + e being at least 2,
f is an integer from 2 to 8,
Z³ is OH, NHR³ or a radical of the formula II
where R³ is hydrogen, C₁- to C₈-alkyl, C₅- to C₈-cycloalkyl or a radical of the formula -(CH₂)ᵤ-NH₂, where u is an integer from 1 to 10,
- polysiloxanes containing repeating units of the formula 1.3 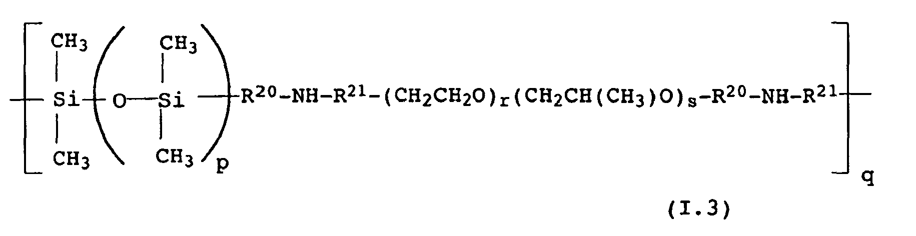 in which
p is an integer from 0 to 100,
q is an integer from 1 to 8,
R²⁰ and R²¹ independently of one another are C₁- to C₈-alkylene,
the order of the alkylene oxide units is arbitrary and
r and s independently of one another are an integer from 0 to 200, the sum r + s being > 0,
- polysiloxanes of the formula I.4 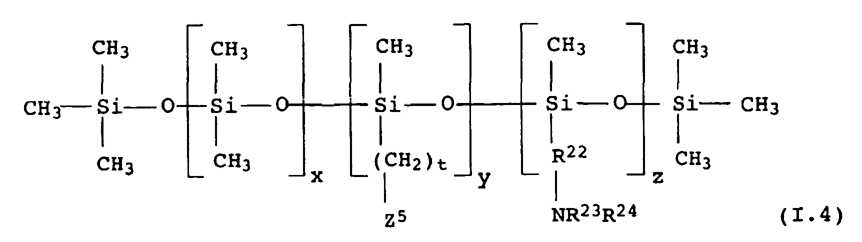 in which
R²² is a C₁- to C₈-alkylene radical,
R²³ and R²⁴ independently of one another are hydrogen, C₁- to C₈-alkyl or C₅- to C₈-cycloalkyl,
the order of the siloxane units is arbitrary,
x, y and z independently of one another are from 0 to 100, the sum x + y + z being at least 3,
t is an integer from 2 to 8,
Z⁵ is a radical of the formula VII
-(OCH₂CH₂)ᵢ(OCH₂CH(CH₃))ⱼ-R²⁵ (VII)
in which
the order of the alkylene oxide units is arbitrary and i and j independently of one another are an integer from 0 to 200, the sum i + j being > 0,
R²⁵ is hydrogen or a C₁- to C₈-alkyl radical,
and mixtures thereof.

4. The composition according to any of the preceding claims, wherein the monomer M) is chosen from
M1) essentially hydrophobic, nonionic compounds, preferably esters of α,β-ethylenically unsaturated mono- and/or dicarboxylic acids with C₁-C₃₀-alkanols, amides of α,β-ethylenically unsaturated mono- and/or dicarboxylic acids with mono- and di-C₁-C₃₀-alkylamines, esters of vinyl alcohol and allyl alcohol with C₁-C₃₀-monocarboxylic acids, vinyl ethers, vinylaromatics, vinyl halides, vinylidene halides, C₂-C₈-monoolefins, nonaromatic hydrocarbons having at least 2 conjugated double bonds and mixtures thereof,
M2) compounds containing a free-radically polymerizable α,β-ethylenically unsaturated double bond and at least one ionogenic and/or ionic group per molecule,
M3) essentially hydrophilic, nonionic compounds, preferably N-vinylamides, N-vinyllactams, primary amides of α,β-ethylenically unsaturated monocarboxylic acids, vinyl- and allyl-substituted heteroaromatic compounds, esters of α,β-ethylenically unsaturated mono- and dicarboxylic acids with C₁-C₃₀-alkanediols, esters and amides of α,β-ethylenically unsaturated mono- and dicarboxylic acids with C₂-C₃₀-amino alcohols which have a primary or secondary amino group, polyether acrylates, and mixtures thereof,
and mixtures thereof.

5. The composition according to any of the preceding claims, wherein the polymer comprises, in copolymerized form,
- from 0.05 to 80% by weight, preferably from 0.1 to 50% by weight, of the urethane (meth)acrylate(s), and
- from 20 to 99.95% by weight, preferably from 50 to 99.9% by weight, of at least one component M).

6. The composition according to any of the preceding claims, wherein the polymer comprises, in copolymerized form,
- from 0.1 to 50% by weight, preferably from 0.5 to 35% by weight, of the urethane (meth)acrylate(s),
- from 40 to 75% by weight, preferably from 45 to 73% by weight, of at least one component M1),
- from 10 to 35% by weight, preferably from 18 to 30%, by weight of at least one component M2),
- from 0 to 30% by weight of at least one component M3).

7. The composition according to any of the preceding claims, wherein the polymer comprises, in copolymerized form,
- from 0.1 to 50% by weight, preferably from 0.5 to 35% by weight, of the urethane (meth)acrylate(s),
- from 0 to 50% by weight of at least one component M1),
- from 0 to 20% by weight of at least one component M2),
- from 25 to 80% by weight of at least one component M3).

8. The hair-treatment composition according to any of the preceding claims, comprising
a) from 0.5 to 20% by weight of at least one water-soluble or -dispersible polymer,
b) from 30 to 99.5% by weight, preferably from 40 to 99% by weight, of at least one solvent chosen from water, water-miscible solvents and mixtures thereof,
c) from 0 bis 70% by weight of a propellant,
d) from 0 to 10% by weight of at least one water-soluble or -dispersible hair polymer which is different from a),
e) from 0 to 0.3% by weight of at least one water-insoluble silicone,
f) from 0 to 1% by weight of at least one nonionic, siloxane-containing, water-soluble or -dispersible polymer.

9. A hair-treatment composition comprising
a) from 0.5 to 20% by weight of at least one water-soluble or -dispersible polymer which comprises, in copolymerized form, at least one siloxane-free urethane (meth)acrylate and at least one free-radically polymerizable α,β-ethylenically unsaturated monomer M), where the siloxane-free urethane (meth)acrylate comprises, in incorporated form, components a), b) and c) as defined in claim 1 and if appropriate at least one other component chosen from components e), f) and g), as defined in claim 2,
b) from 30 to 99.5% by weight, preferably from 40 to 99% by weight, of at least one solvent chosen from water, water-miscible solvents and mixtures thereof,
c) from 0 to 70% by weight of a propellant,
d) from 0 to 10% by weight of at least one water-soluble or -dispersible hair polymer which is different from a),
e) from 0 to 0.3% by weight of at least one water-insoluble silicone,
f) from 0 to 1% by weight of at least one nonionic, siloxane-containing, water-soluble or -dispersible polymer.

10. The use of water-soluble or water-dispersible polymers chosen from polymers as defined in any of claims 1 to 7, and mixtures thereof in hair cosmetics, preferably as setting polymers in hairsprays, setting foams, hair mousse, hair gel and shampoos, and in skin cosmetics, preferably in creams, pigment-containing skin cosmetics and wax-containing skin cosmetics.

11. A free-radically polymerizable, siloxane-containing urethane (meth)acrylate as defined in any of claims 1 to 3, which comprises, in incorporated form, tert-butylaminoethyl acrylate and/or tert-butylaminoethyl methacrylate as component a), or a salt therof.

12. A water-soluble or water-dispersible polymer which comprises, in copolymerized form, at least one urethane (meth)acrylate as claimed in claim 11 and at least one free-radically polymerizable α,β-ethylenically unsaturated monomer M).

## Revendications

1. Produit de traitement des cheveux, qui contient au moins un polymère soluble ou dispersible dans l'eau ainsi que des adjuvants cosmétiques courants, le polymère contenant à l'état copolymérisé au moins un (méth)acrylate d'uréthanne et au moins un monomère M) α,β-éthyléniquement insaturé polymérisable par voie radicalaire, le (méth)acrylate d'uréthanne étant choisi parmi des (méth)acrylates d'uréthanne polymérisables par voie radicalaire, qui contiennent des groupes siloxane et, sous forme incorporée,
a) au moins un composé qui contient, par molécule, au moins un atome d'hydrogène actif et au moins une double liaison α,β-éthyléniquement insaturée, polymérisable par voie radicalaire,
b) au moins un diisocyanate,
c) au moins un composé qui contient, par molécule, deux atomes d'hydrogène actifs,
d) au moins un composé qui contient, par molécule, au moins un atome d'hydrogène actif et au moins un groupe siloxane,
et les sels de ces substances.

2. Produit suivant la revendication 1, dans lequel les (méth)acrylates d'uréthanne contiennent en supplément, sous forme incorporée, au moins un composant qui est choisi parmi
e) des composés qui contiennent, par molécule, deux ou plusieurs atomes d'hydrogène actifs et au moins un groupe ionogène et/ou ionique,
f) des monoalcools, des amines comportant un groupe amino primaire ou secondaire, des monoisocyanates aliphatiques, cycloaliphatiques ou aromatiques, et leurs mélanges,
g) des composés α,β-éthyléniquement insaturés qui contiennent en supplément, par molécule, au moins un groupe isocyanate,
et leurs mélanges.

3. Produit suivant l'une des revendications 1 et 2, dans lequel le composant d) est choisi parmi
- des polysiloxanes de la formule générale I.1 : 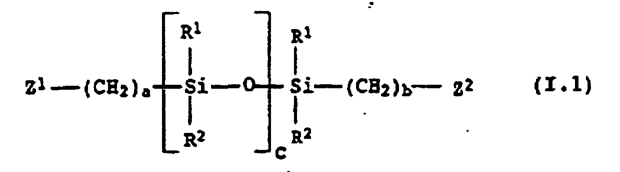 dans laquelle
a et b valent indépendamment l'un de l'autre 2 à 8,
c vaut 3 à 100,
R¹ et R² représentent, indépendamment l'un de l'autre, un groupe alkyle en C₁-C₈, benzyle ou phényle,
Z¹ et Z² représentent, indépendamment l'un de l'autre, OH, NHR³ ou un radical de la formule II :
-O-(CH₂CH₂O)ᵥ(CH₂CH(CH₃)O)_{w}-H (II)
où la succession des unités d'oxyde d'alkylène est quelconque dans la formule II, et v et w représentent
indépendamment l'un de l'autre un nombre entier de 0 à 200, la somme de v et w étant> 0,
R³ représente de l'hydrogène ou un groupe alkyle en C₁-C₈ ou cycloalkyle en C₅-C₈,
- des polysiloxanes de la formule générale I.2 : 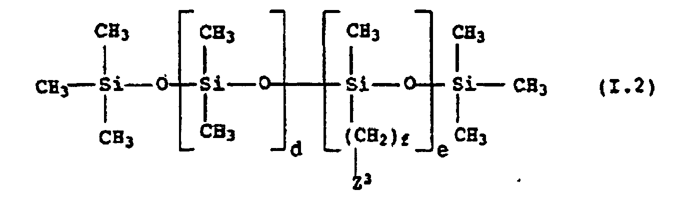 dans laquelle la succession des unités de siloxane est quelconque,
d et e représentent indépendamment l'un de l'autre, 0 à 100, la somme de d et e étant d'au moins 2,
f représente un nombre eutier de 2 à 8,
Z³ représente OH, NHR³ ou un radical de la formule II,
R³ représentant de l'hydrogène ou un groupe alkyle en C₁-C₈, cycloalkyle en C₅-C₈, ou un radical de la formule -(CH₂)ᵤ-NH₂, où u représente un nombre entier de 1 à 10,
- des polysiloxanes comportant des unités répétitives de la formule générale I.3 : 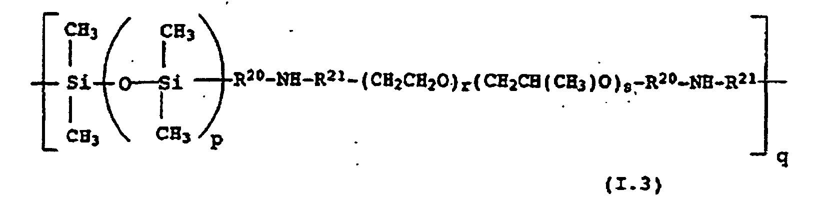 dans laquelle
p représente un nombre entier de 0 à 100,
q représente un nombre entier de 1 à 8,
R²⁰ et R²¹ représentent, indépendamment l'un de l'autre, un groupe alkylène en C₁-C₈,
la succession des unités d'oxyde d'alkylène est quelconque, et
r et s représentent, indépendamment l'un de l'autre, un nombre entier de 0 à 200, la somme de r et s étant > 0,
- des polysiloxanes de la formule générale 1.4 : 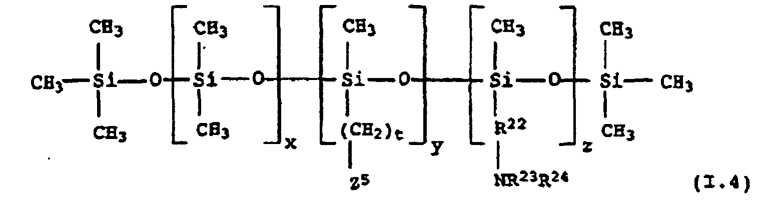 dans laquelle
R²² représente un radical alkylène en C₁-C₈,
R²³ et R²⁴ représentent, indépendamment l'un de l'autre, de l'hydrogène ou un groupe alkyle en C₁-C₈ ou cycloalkyle en C₅-C₈,
la succession des unités de siloxane est quelconque,
x, y et z représentent, indépendamment l'un de l'autre, une valeur de 0 à 100, la somme de x, y et z étant d'au moins 3,
t représente un nombre entier de 2 à 8,
Z⁵ représente un radical de la formule VII :
-(OCH₂CH₂)ᵢ(OCH₂CH(CH₃))ⱼ-R²⁵ (VII)
où la succession des unités d'oxyde d'alkylène est quelconque et i et j représentent, indépendamment l'un de l'autre, un nombre entier de 0 à 200, la somme de i et j étant> 0,
R²⁵ représente de l'hydrogène ou un radical alkyle en C₁-C₈,
et leurs mélanges.

4. Produit suivant l'une des revendications précédentes, dans lequel le monomère M) est choisi parmi
M1) des composés non ioniques essentiellement hydrophobes, de préférence des esters d'acides monocarboxyliques et/ou dicarboxyliques α,β-éthyléniquement insaturés avec des alcanols en C₁-C₃₀, des amides d'acides monocarboxyliques et/ou dicarboxyliques α,β-éthyléniquement insaturés avec des monoalkylamines et dialkylamines en C₁-C₃₀, des esters d'alcool vinylique et d'alcool allylique avec des acides monocarboxyliques en C₁-C₃₀, des éthers vinyliques, des substances vinylaromatiques, des halogénures de vinyle, des halogénures de vinylidène, des monooléfines en C₂-C₈, des hydrocarbures non aromatiques comportant au moins deux doubles liaisons conjuguées et leurs mélanges,
M2) des composés comportant une double liaison α,β-éthyléniquement insaturée polymérisable par voie radicalaire, et au moins un groupe ionogène et/ou ionique par molécule,
M3) des composés non ioniques, essentiellement hydrophiles, de préférence des N-vinylamides, des N-vinyllactames, des amides primaires d'acides monocarboxyliques α,β-éthyléniquement insaturés, des composés hétéroaromatiques substitués par du vinyle et de l'allyle, des esters d'acides monocarboxyliques et dicarboxyliques α,β-éthyléniquement insaturés avec des alcanediols en C₁-C₃₀, des esters et des amides d'acides monocarboxyliques et dicarboxyliques α,β-éthyléniquement insaturés avec des aminoalcools en C₂-C₃₀, qui présentent un groupe amino primaire ou secondaire, des polyétheracrylates et leurs mélanges,
et des mélanges de ces substances.

5. Produit suivant l'une des revendications précédentes, dans lequel le polymère contient, à l'état copolymérisé,
- 0,05 à 80 % en poids, de préférence 0,1 à 50 % en poids, du ou des (méth)acrylates d'uréthanne, et
- 20 à 99,95 % en poids, de préférence 50 à 99,9 % en poids, d'au moins un composant M).

6. Produit suivant l'une des revendications précédentes, dans lequel le polymère contient, à l'état copolymérisé,
- 0,1 à 50 % en poids, de préférence 0,5 à 35 % en poids, du ou des (méth)acrylates d'uréthanne,
- 40 à 75 % en poids, de préférence 45 à 73 % en poids, d'au moins un composant M1),
- 10 à 35 % en poids, de préférence 18 à 30 % en poids, d'au moins un composant M2),
- 0 à 30 % en poids d'au moins un composant M3).

7. Produit suivant l'une des revendications précédentes, dans lequel le polymère contient, à l'état copolymérisé,
- 0,1 à 50 % en poids, de préférence 0,5 à 35 % en poids, du ou des (méth)acrylates d'uréthanne,
- 0 à 50 % en poids d'au moins un composant M1),
- 0 à 20 % en poids d'au moins un composant M2),
- 25 à 80 % en poids d'au moins un composant M3).

8. Produit de traitement des cheveux suivant l'une des revendications précédentes, contenant
a) 0,5 à 20 % en poids d'au moins un polymère soluble ou dispersible dans l'eau,
b) 30 à 99,5 % en poids, de préférence 40 à 99 % en poids, d'au moins un solvant choisi parmi de l'eau, des solvants miscibles à l'eau et leurs mélanges,
c) 0 à 70 % en poids d'un agent gonflant,
d) 0 à 10 % en poids d'au moins un polymère pour les cheveux soluble ou dispersible dans l'eau, différent de a),
e) 0 à 0,3 % en poids d'au moins une silicone insoluble dans l'eau,
f) 0 à 1 % en poids d'au moins un polymère soluble ou dispersible dans l'eau, contenant du siloxane, non ionique.

9. Produit de traitement des cheveux contenant
a) 0,5 à 20 % en poids d'au moins un polymère soluble ou dispersible dans l'eau qui contient, à l'état copolymérisé, au moins un (méth)acrylate d'uréthanne exempt de groupes siloxane et au moins un monomère M) α,β-éthyléniquement insaturé, polymérisable par voie radicalaire, le (méth)acrylate d'uréthanne exempt de groupes siloxane contenant, sous forme incorporée, les composés a), b) et c), tels que définis dans la revendication 1, et éventuellement au moins un autre composant qui est choisi parmi les composants e), f) et g), tels que définis dans la revendication 2,
b) 30 à 99,5 % en poids, de préférence 40 à 99 % en poids d'au moins un solvant choisi parmi de l'eau, des solvants miscibles à l'eau et leurs mélanges,
c) 0 à 70 % en poids d'un agent gonflant,
d) 0 à 10 % en poids d'au moins un polymère pour les cheveux soluble ou dispersible dans l'eau, différent de a),
e) 0 à 0,3 % en poids d'au moins une silicone insoluble dans l'eau,
f) 0 à 1 % en poids d'au moins un polymère soluble ou dispersible dans l'eau, contenant du siloxane, non ionique.

10. Utilisation de polymères solubles ou dispersibles dans l'eau qui sont choisis parmi des polymères tels que définis dans l'une des revendications 1 à 7 et leurs mélanges, dans le domaine des cosmétiques pour les cheveux, de préférence sous la forme d'un polymère renforçateur dans des pulvérisations pour cheveux, des renforçateurs mousse, des mousses capillaires, des gels pour les cheveux et des shampooings, et dans le domaine des cosmétiques pour la peau, de préférence dans des crèmes, des produits cosmétiques pour la peau contenant des pigments et des produits cosmétiques pour la peau contenant de la cire.

11. (Méth) acrylates d'uréthanne contenant des groupes siloxane, polymérisables par voie radicalaire, tels que définis dans l'une des revendications 1 à 3, qui contiennent, sous forme incorporée, comme composant a), de l'acrylate de tert-butylaminoéthyle et/ou du méthacrylate de tert-butylaminoéthyle, et les sels de ces substances.

12. Polymère soluble ou dispersible dans l'eau, qui contient, à l'état copolymérisé, au moins un (méth)acrylate d'uréthanne suivant la revendication 11 et au moins un monomère M) α,β-éthyléniquement insaturé, polymérisable par voie radicalaire.
